(19) Europäisches Patentamt
European Patent Office
Office européen des brevets

(11) **EP 3 399 427 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(51) Int Cl.:
**G06F 17/18** (2006.01)          **A61B 5/11** (2006.01)
**A61B 5/18** (2006.01)          **G06F 3/01** (2006.01)
**A61B 5/16** (2006.01)          **G06F 3/00** (2006.01)

(21) Anmeldenummer: **17169254.4**

(22) Anmeldetag: **03.05.2017**

(54) **VERFAHREN UND SYSTEM ZUR QUANTITATIVEN MESSUNG DER MENTALEN BEANSPRUCHUNG EINES INDIVIDUELLEN BENUTZERS**

METHOD AND SYSTEM FOR THE QUANTITATIVE MEASUREMENT OF MENTAL STRESS OF AN INDIVIDUAL USER

PROCÉDÉ ET SYSTÈME DE MESURE QUANTITATIVE DE SOLLICITATION INTELLECTUELLE D'UN UTILISATEUR INDIVIDUEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**07.11.2018 Patentblatt 2018/45**

(73) Patentinhaber: **Karlsruher Institut für Technologie**
**76131 Karlsruhe (DE)**

(72) Erfinder: **Schneider, Marc**
**76133 Karlsruhe (DE)**

(74) Vertreter: **Bittner, Peter et al**
**Peter Bittner und Partner**
**Herrenwiesenweg 2**
**69207 Sandhausen (DE)**

(56) Entgegenhaltungen:
**WO-A1-2014/159793     US-A1- 2007 300 185**

- **CONATI ET AL: "Eye-tracking for user modeling in exploratory learning environments: An empirical evaluation", KNOWLEDGE-BASED SYSTEMS, ELSEVIER, AMSTERDAM, NL, Bd. 20, Nr. 6, 19. Juli 2007 (2007-07-19), Seiten 557-574, XP022163628, ISSN: 0950-7051, DOI: 10.1016/J.KNOSYS.2007.04.010**
- **GOLDBERG J H ET AL: "Computer interface evaluation using eye movements: Methods and constructs", INTERNATIONAL JOURNAL OF INDUSTRIAL ERGONOMICS, ELSEVIER, AMSTERDAM, NL, Bd. 24, 1. Januar 1999 (1999-01-01), Seiten 631-645, XP002375287, ISSN: 0169-8141, DOI: 10.1016/S0169-8141(98)00068-7**
- **SCHWARZ JESSICA ET AL: "Towards a more holistic view on user state assessment in adaptive human-computer interaction", 2014 IEEE INTERNATIONAL CONFERENCE ON SYSTEMS, MAN, AND CYBERNETICS (SMC), IEEE, 5. Oktober 2014 (2014-10-05), Seiten 1228-1234, XP032693859, DOI: 10.1109/SMC.2014.6974082 [gefunden am 2014-12-03]**

**Beschreibung**

**Technisches Gebiet**

Die Erfindung bezieht sich auf elektronische Datenverarbeitung im Allgemeinen und im Speziellen auf ein Computersystem, Computerprogrammprodukt und computerimplementierte Verfahren zur Bestimmung eines geeigneten Messverfahrens für die quantitative Messung der mentalen Beanspruchung eines individuellen Benutzers bei dessen Interaktion mit einem technischen System sowie der Ausführung des Messverfahrens.

**Stand der Technik**

Bei der Interaktion eines menschlichen Benutzers mit technischen Systemen über entsprechende Benutzerschnittstellen kann der individuelle Benutzer unter Umständen einer hohen mentalen Beanspruchung ausgesetzt sein, wenn die Benutzerschnittstellen nicht für das jeweilige Individuum optimiert sind. Eine hohe mentale Beanspruchung kann über einen längeren Zeitraum zu einer permanenten Überforderung und zu einer gesundheitlichen Schädigung des Benutzers führen.

Zur Verbesserung der Benutzerschnittstellen werden im Stand der Technik Verfahren eingesetzt, die eine Abschätzung der mentalen Beanspruchung nach dem Prinzip "one fits all" vornehmen. Dabei wird üblicherweise ein Beanspruchungsparameter bestimmt, der dann unter der Annahme, dass er auf alle individuellen Benutzer mit dem gleichen Ergebnis anwendbar ist, als Grundlage für die Optimierung der Benutzerschnittstellen Verwendung findet. Allerdings ist die Beanspruchung sehr individuell und verschiedene Benutzer zeigen deutlich unterschiedliche Reaktionen bei verschiedenen Beanspruchungen. Deshalb können die bekannten Verfahren leicht in Benutzerschnittstellen resultieren, bei denen einzelne Benutzer häufig in bestimmten Situationen überfordert werden, was dann die oben genannten Nachteile nach sich zieht. Die PCT Anmeldung WO 2014/159793 A1 beschreibt ein Stand der Technik Verfahren zum Schätzen des Benutzerzustands in Echtzeit basierend unter anderem auf blickbasierten Beanspruchungsparametern. Mittels eines "machine learning" Algorithmus und eines Hidden Markov Modells wird ein Schätzmodell für den Benutzerzustand bestimmt.

Es besteht daher die Notwendigkeit, ein quantifiziertes Messverfahren für die mentale Beanspruchung eines individuellen Benutzers, sowie ein entsprechendes System dafür bereitzustellen. Mit einem individualisierten Messverfahren kann dann die Benutzerschnittstelle auf den individuellen Benutzer angepasst werden, um seine mentale Beanspruchung während der Interaktion mit dem technischen System zu minimieren.

**Überblick**

Das technische Problem der quantitativen Messung der mentalen Beanspruchung eines individuellen Benutzers während seiner Interaktion mit einem technischen System wird durch ein computerimplementiertes Verfahren, ein Computersystem, sowie ein entsprechendes Computerprogrammprodukt mit den in den unabhängigen Ansprüchen beschriebenen Merkmalen gelöst. In den Unteransprüchen werden bevorzugte Ausführungsformen beschrieben.

In einer Ausführungsform wird ein computerimplementiertes Verfahren zur quantitativen Messung der mentalen Beanspruchung eines individuellen Benutzers bei dessen Interaktion mit einem technischen System bereitgestellt. Das Verfahren umfasst die im Folgenden beschriebenen Schritte a) bis g):

a) Empfangen von einzelnen Parametersignalen für mehrere blickbasierte Beanspruchungsparameter des individuellen Benutzers, die während der Bearbeitung mehrerer Kalibrierungsaufgaben über jeweils zwei oder mehr Kalibrierungszeitintervalle hinweg erfasst werden, wobei die Kalibrierungsaufgaben zwei oder mehr verschiedene Aufgabenarten mit jeweils zwei oder mehr verschiedenen Anforderungsniveaus umfassen.

Blickbasierte Beanspruchungsparametersignale können mittels handelsüblicher Blickerfassungssysteme erfasst werden, wie sie beispielsweise von den Anbietern Ergoneers GmbH, Manching, Deutschland und Pupil Labs UG, Berlin, Deutschland erhältlich sind. Solche Blickerfassungssysteme beinhalten üblicherweise eine eye-tracking Lösung mit entsprechenden Softwarepaketen, die das Messen bestimmter Parameter, wie beispielsweise Blinzelrate oder Pupillendurchschnittsvariabilität, ermöglichen. Weitere Parameter werden in der Detailbeschreibung näher erörtert. Beispielsweise können manche Beanspruchungsparameter einen Indikator für einen bewussten Verarbeitungsprozess repräsentieren. Solche "bewussten Parameter" können dabei einer der folgenden bewussten Parametergruppen zugeordnet sein: Fixationsdauer, Sakkadenlänge, Nearest Neighbor Index. Beanspruchungsparameter, die einen Indikator für einen unbewussten Verarbeitungsprozess repräsentieren können dagegen einer der folgenden bewussten Parametergruppen zugeordnet sein: Blinzelrate, Blinzeldauer, PERCLOS, Pupillendurchschnittsvariabilität.

Eine Kalibrierungsaufgabe besteht üblicherweise aus einer Hauptaufgabe und einer Nebenaufgabe. Die Nebenaufgaben können dabei arithmetische und/oder visuelle Aufgaben umfassen. Die mentale Beanspruchung beschreibt die kognitive Reaktion des menschlichen Informationsverarbeitungssystems auf äußere Belastungen in Abhängigkeit von seinen persönlichen Vorrausetzungen und den individuellen Bewältigungsstrategien (Packebusch, L. (2003). Psychische Belastung und Beanspruchung - Normung für die Praxis. In Wirtschaftspsychologie aktuell (Bd. 4, S. 32 - 36). Deutscher Psychologen Verlag GmbH; Niederl, T. (2007). Untersuchung zu kumulativen psychischen und physiologischen Effekten des fliegerischen Personals auf der Kurzstrecke. Forschungsbericht des Deutschen Zentrum für Luft- und Raumfahrt e. V. 17).

b) Ermitteln von normierten Leistungsdaten als einzelne Referenzsignale für die jeweiligen Aufgaben aus den einzelnen Parametersignalen, wobei die Leistungsdaten ein Maß für die mentale Beanspruchung während der Bearbeitung der Kalibrierungsaufgaben darstellen.

Die mentale Beanspruchung während des Bearbeitens der Kalibrierungsaufgaben kann z.B. über leistungsbasierte Parameter (Fehler in Haupt- und Nebenaufgaben) bestimmt werden. Weiterhin liefern auch physiologische Reaktionen des Menschen (Okulometrie, EKG, EDA), und sein subjektives Empfinden (mittels Fragebögen) ergänzende Informationen zur mentalen Beanspruchung (Manzey, D. (1998). Psychophysiologie mentaler Beanspruchung. In F. Rößler (Hrsg.), Enyklopädie der Psychologie (Bd. 5, S. 799-864). Göttingen: Hogrefe). In anderen Worten, die Leistungsdaten entsprechend einem Prozentsatz, der beschreibt, wie gut die entsprechende Kalibrierungsaufgabe vom Benutzer gelöst wurde. Detaillierte Beispiele sind in der Detailbeschreibung offenbart. Im Falle visueller Nebenaufgaben können vom System zusätzlich zu den Leistungsdaten auch die Reaktionszeiten erfasst werden und für die Ermittlung der Referenzsignale verwendet werden.

c) Auswählen der Beanspruchungsparameter, deren Gesamtparametersignale eine höhere Ähnlichkeit zum Gesamtreferenzsignal aufweisen als die Ähnlichkeit ihrer einzelnen Parametersignale zu den jeweiligen einzelnen Referenzsignalen für die einzelnen Ausprägungen von Aufgabenart, Anforderungsniveau und Zeitintervall. Das Ergebnis eines solchen Filterschritts liefert nur noch solche Beanspruchungsparameter, die sich für den individuellen Benutzer als unabhängig von Aufgabenart, Aufgabenanforderung und Aufgabendauer erweisen.

Weiterhin umfasst das Verfahren die folgenden Schritte: Erzeugen des Gesamtreferenzsignals aus den einzelnen Referenzsignalen sowie der Gesamtparametersignale aus den einzelnen Parametersignalen für jeden erfassten Beanspruchungsparameter durch Aneinanderreihen der jeweiligen Messergebnisse für alle Teilaufgaben der Kalibrierungsaufgaben; Ermitteln einer Ähnlichkeit für jedes der Gesamtparametersignale mit dem Gesamtreferenzsignal über eine Procrustes-Analyse. In einer Ausführungsform kann das Auswählen der Beanspruchungsparameters mittels der Procrustes-Analyse mit den folgenden Schritten erfolgen: Aufteilen der Gesamtparametersignale und des Gesamtreferenzsignals nach den Ausprägungen der Aufgabenarten, der Anforderungsniveaus und der einzelnen Zeitintervalle; Durchführen der Procrustes-Analyse für jede Ausprägung, zwischen den einzelnen Parametersignalen und den einzelnen Referenzsignalen; und Vergleichen der Ähnlichkeiten der einzelnen Parametersignale zu den einzelnen Referenzsignalen mit der Ähnlichkeit der Gesamtparametersignale zum Gesamtreferenzsignal.

d) Ermitteln von mindestens einem drift-freien Beanspruchungsparameters mit der höchsten Adaptionslatenz unter den ausgewählten Beanspruchungsparametern. Hierfür kann zunächst für jeden gefilterten Beanspruchungsparameter eine Adaptionslatenz z.B. mittels einer Moving-Correlation-Analyse bestimmt werden. Darauf wird die mittlere Adaptionslatenz von bewussten Beanspruchungsparametern mit der mittleren Adaptionslatenz von unbewussten Beanspruchungsparametern verglichen und schließlich können die gefilterten Beanspruchungsparameter der Kategorie mit der höheren mittleren Adaptionslatenz (d.h. der bewußten oder der unbewussten Parameter) selektiert werden. Bewusste Beanspruchungsparameter repräsentieren einen Indikator für einen bewussten Verarbeitungsprozess und sind üblicherweise einer der folgenden bewussten Parametergruppen zugeordnet: Fixationsdauer, Sakkadenlänge, oder Nearest Neighbor Index. Unbewussten Beanspruchungsparameter repräsentieren einen Indikator für einen unbewussten Verarbeitungsprozess und sind üblicherweise einer der folgenden unbewussten Parametergruppen zugeordnet: Blinzelrate, Blinzeldauer, PERCLOS, Pupillendurchschnittsvariabilität. In jeder Parametergruppe kann es eine Vielzahl von einzelnen Beanspruchungsparametern geben, die in der Detailbeschreibung näher ausgeführt werden.

Unter den selektierten Beanspruchungsparametern können dann drift-freie Beanspruchungsparameter bestimmt werden, die im Betrachtungsbereich des Referenzsignals nicht von dem Referenzsignal abdriften. Schließlich kann daraus der drift-freie Beanspruchungsparameter mit der höchsten Adaptionslatenz ermittelt werden.

e) Generieren einer individuellen Beanspruchungsfunktion für den Benutzer durch Überführen von mindestens einem der ermittelten drift-freien Beanspruchungsparameter in eine individuelle lineare Funktion mittels linearer Regression aus den während der Kalibrierungsaufgabe für den mindestens einen ermittelten drift-freien Beanspruchungsparameter erfassten Leistungsdaten.

Mit dem letzten Schritt ist das quantifizierte Messverfahren für die mentale Beanspruchung eines individuellen Benutzers eindeutig bestimmt. Der ermittelte drift-freie Beanspruchungsparameter ist der für den individuellen Benutzer der am besten geeignete Parameter für die spätere Messung der mentalen Beanspruchung während der Interaktion mit einem technischen System. Ein Vorteil des oben beschriebenen Verfahrens liegt in der Unabhängigkeit des ermittelten Parameters von der Aufgabenart, dem Anforderungsniveau und der Dauer der Beanspruchung. Deshalb ist der ermittelte Parameter geeignet, die mentale Beanspruchung des Benutzers bei der Interaktion mit einem beliebigen technischen System zu messen. Die generierte Beanspruchungsfunktion liefert dabei ein Werkzeug, das aus den gemessenen Beanspruchungsparametersignalen direkt die entsprechenden Leistungsdaten für den Benutzer bestimmt.

Das nun eindeutig bestimmte Messverfahren wird im Anschluss für das erfindungsgemäße computerimplementierte Verfahren zur quantitativen Messung der mentalen Beanspruchung des individuellen Benutzers bei dessen Interaktion mit einem technischen System eingesetzt Die Messung umfasst dabei die folgenden Schritte f) und g):

f) Erfassen des mindestens einen ermittelten drift-freien Beanspruchungsparameters während der Interaktion des individuellen Benutzers mit dem technischen System über mehrere Messzeitintervalle. Das Erfassen des ermittelten Parameters (d.h., der jeweiligen Parametersignale) erfolgt dabei mit dem bereits zuvor während der Bestimmung des Messverfahrens (Kalibrierung) eingesetzten Blickerfassungssystems.

g) Ableiten der Leistungsdaten pro Messzeitintervall als mentale Beanspruchung mittels der individuellen Beanspruchungsfunktion. Dabei werden lediglich die Messwerte aus Schritt f) in die generierte Beanspruchungsfunktion eingesetzt und damit die entsprechenden Leistungsdaten berechnet.

Um die Interaktion des individuellen Benutzers mit dem technischen System zu optimieren, ist es insbesondere nützlich, seine mentale Beanspruchung in Abhängigkeit einer Blickstrategie zu bestimmen. Beispielsweise ändert sich üblicherweise die Blickstrategie eines Benutzers, der z.B. mit einem Navigationssystem während einer Autofahrt interagiert, wenn der Benutzer von der Autobahn auf eine Landstraße abfährt und danach in einen Innerortsbereich einfährt. Der Fokus der Aufmerksamkeit des Benutzers bei einer Autobahnfahrt wird in der Regel gerade aus auf den vorausfahrenden Verkehr gerichtet sein. Innerorts dagegen wird der Fokus häufig auf die Seite der Fahrbahn gerichtet sein (parkende Autos, spielende Kinder, etc.). Eine immer gleiche Benutzerschnittstelle des Navigationssystems kann zu deutlich unterschiedlichen Beanspruchungsniveaus für die verschiedenen Blickstrategien führen, was letztendlich in einer verkehrsgefährdenden Situation resultieren kann, wenn eine zu große Überbeanspruchung entsteht.

Das zuvor bestimmte Messverfahren lässt sich mit den Schritten h) bis k) noch weiter verfeinern, um die Abhängigkeit von der Blickstrategie zu berücksichtigen:

h) Erfassen von Fixationskoordinaten, Fixationshäufigkeit und Fixationsdauer des individuellen Benutzers während der Interaktion mit dem technischen System. Die Fixationsparameter können wiederum mit dem benutzten Blickerfassungssystem (eye tracker) gemessen werden.

i) Ermitteln von Indikatoren für die Blickstrategie des individuellen Benutzers mit einer Kombination aus einer Clusteranalyse der Fixationskoordinaten und einer Analyse der Fixationshäufigkeit und -dauer;

j) Bestimmen der Grenzen der Messzeitintervalle als Zeitpunkte von Blickstrategiewechseln mittels Veränderungen in den Ergebnissen der Clusteranalyse der Fixationskoordinaten oder der Analyse von Fixationshäufigkeit und Fixationsdauer.

k) Ableiten der mentalen Beanspruchung des individuellen Benutzers in Abhängigkeit der angewandten Blickstrategie.

In einer Ausführungsform wird ein Computerprogrammprodukt bereitgestellt, welches computerlesbare Instruktionen umfasst, die beim Laden in mindestens ein Speichermodul eines Computers und beim Abarbeiten durch mindestens einen Prozessor des Computers die hier beschriebenen computerimplementierten Verfahren ausführen.

In einer weiteren Ausführungsform wird ein Computersystem zur quantitativen Messung der mentalen Beanspruchung eines individuellen Benutzers bei dessen Interaktion mit einem technischen System bereitgestellt. Das Computersystem umfasst Speicher- und Prozessoreinheiten die das besagte Computerprogrammprodukt ausführen können und wodurch die im Folgenden kurz beschriebenen Module implementiert werden:

Ein erstes Erfassungsmodul konfiguriert zum Empfangen von einzelnen Parametersignalen für mehrere blickbasierte Beanspruchungsparameter des individuellen Benutzers, die während der Bearbeitung mehrerer Kalibrierungsaufgaben über jeweils zwei oder mehr Kalibrierungszeitintervalle hinweg erfasst werden, wobei die Kalibrierungsaufgaben zwei oder mehr verschiedene Aufgabenarten mit jeweils zwei oder mehr verschiedenen Anforderungsniveaus umfassen.

Ein Auswertemodul ist konfiguriert zum Ermitteln von normierten Leistungsdaten als einzelne Referenzsignale für die jeweiligen Aufgaben aus den einzelnen Parametersignalen, wobei die Leistungsdaten ein Maß für die mentale Beanspruchung des Benutzers während der Bearbeitung der Kalibrierungsaufgaben darstellen. Weiterhin ist es konfiguriert zum Erzeugen eines Gesamtreferenzsignals aus den einzelnen Referenzsignalen sowie von Gesamtparametersignalen aus den einzelnen Parametersignalen für jeden erfassten Beanspruchungsparameter durch aneinanderreihen der jeweiligen Messergebnisse für alle Teilaufgaben der Kalibrierungsaufgaben; zum Auswählen der Beanspruchungsparameter, deren Gesamtparametersignale eine höhere Ähnlichkeit zum Gesamtreferenzsignal aufweisen als die Ähnlichkeit ihrer einzelnen Parametersignale zu den jeweiligen einzelnen Referenzsignalen für die einzelnen Ausprägungen von Aufgabenart, Anforderungsniveau und Zeitintervall, wobei die jeweiligen Ähnlichkeiten jeweils über die Procrustes-Analyse ermittelt werden; zum Ermitteln von mindestens einem drift-freien Beanspruchungsparameter mit der höchsten Adaptionslatenz unter den ausgewählten Beanspruchungsparametern; und zum Generieren einer individuellen Beanspruchungsfunktion für den Benutzer durch Überführen von mindestens einem der ermittelten drift-freien Beanspruchungsparameter in eine individuelle lineare Funktion mittels linearer Regression aus den während der Kalibrierungsaufgabe für den mindestens einen der ermittelten drift-freien Beanspruchungsparameter erfassten Leistungsdaten.

Das Computersystem wird zu einem Computersystem zur quantitativen Messung der mentalen Beanspruchung des individuellen Benutzers bei dessen Interaktion mit einem technischen System ergänzt, in dem weitere Module hinzugefügt werden. Diese weiteren Module umfassen:

Ein zweites Erfassungsmodul konfiguriert zum Erfassen des mindestens einen ermittelten drift-freien Beanspruchungsparameters während der Interaktion des individuellen Benutzers mit dem technischen System über mehrere Messzeitintervalle; und ein Analysemodul zum Ableiten der Leistungsdaten pro Messzeitintervall als mentale Beanspruchung mittels der individuellen Beanspruchungsfunktion.

Optional kann das zweite Erfassungsmodul weiterhin zum Erfassen von Fixationskoordinaten, Fixationshäufigkeit und Fixationsdauer des individuellen Benutzers während der Interaktion mit dem technischen System konfiguriert werden. Das Analysemodul kann dabei weiterhin konfiguriert werden zum: Ermitteln von Indikatoren für die Blickstrategie des individuellen Benutzers mit einer Kombination aus einer Clusteranalyse der Fixationskoordinaten und einer Analyse der Fixationshäufigkeit und Fixationsdauer; Bestimmen der Grenzen der Messzeitintervalle als Zeitpunkte von Blickstrategiewechseln mittels Veränderungen in den Ergebnissen der Clusteranalyse der Fixationskoordinaten oder der Analyse von Fixationshäufigkeit und -dauer; und Ableiten der mentalen Beanspruchung des individuellen Benutzers in Abhängigkeit der angewandten Blickstrategie.

Weitere vorteilhafte Ausführungsformen der Erfindung finden sich in den abhängigen Ansprüchen wieder.

Bei der Interaktion von Menschen mit technischen Systemen (z.B. Anlagen, Fahrzeugen, Flugzeugen, Leitwarten) kommt es häufig zu unsicheren Situation oder zu Unfällen, die auf eine unzureichende Systemgestaltung zurückzuführen sind (Sträter, O. (2005). Cognition and Safety: An integrated approach to systems design and assessment. Aldershot, England and Burlington, VT: Ashgate). Der Mensch wird durch das System oder die bereitgestellten Informationen fehlgeleitet, findet in kritischen Situationen die relevanten Informationen nur schwer oder wird in bestimmten Situationen nicht optimal beansprucht. Die erfindungsgemäße zuverlässige Bewertung (Messung) der mentalen Beanspruchung unter Berücksichtigung aktueller Strategien der individuellen Benutzer ermöglicht die direkte Ableitung von Gestaltungsempfehlungen aus dem Verhalten und den Bedürfnissen der Nutzer heraus. Durch die integrierte Analyse von Beanspruchungen und Strategien ergibt sich die Möglichkeit, sichere, zuverlässige sowie beanspruchungsoptimierte technische Systeme zu schaffen. Die zeitabhängige Analyse von Über- und Unterbeanspruchungen der Nutzer kann für eine systemgestalterische Fehlerprävention und eine Optimierung der menschlichen Energiebilanz genutzt werden. Die Untersuchung von Veränderungen der Blickstrategie sowie deren Auswirkungen auf die Beanspruchung ermöglicht die Identifikation von Ablenkungseffekten, die durch gezielte Designempfehlungen abgefangen werden können. So kann durch die Erfindung für spezifische Interaktionsszenarien ein in sich konsistentes, adaptives und/oder erwartungskonformes Interface- und Schnittstellendesign realisiert werden. Schließlich liefern die generierten Daten eine der Voraussetzungen für eine adaptive Systemgestaltung. Durch die Analyse der Veränderungen in den individuellen Strategien kann die Verhaltensvariabilität der Nutzer und deren Auswirkung auf den Beanspruchungszustand erfasst werden, was die klare Identifikation von Adaptionszeitpunkten und Adaptionsrichtungen zulässt.

**Kurzbeschreibung der Zeichnungen**

FIG. 1 zeigt ein Ausführungsbeispiel eines Computersystems zur Bestimmung eines geeigneten Verfahrens für die quantitative Messung der mentalen Beanspruchung und ein erweitertes Ausführungsbeispiel zur Durchführung des Verfahrens;

FIG. 2A - 2D zeigen Flussdiagramme zur Veranschaulichung der erfindungsgemäßen Verfahren;

FIG. 3A, 3B zeigen ein Beispiel für ein mögliches Layout einer Kalibrierungsaufgabe;

FIG. 4A zeigt Beispiele für eine gemessene und eine prognostizierte Beanspruchungsfunktion für das Referenzsignal Leistung;

FIG. 4B zeigt Beispiele für eine gemessene und eine prognostizierte Beanspruchungsfunktion für das Referenzsignal subjektives Empfinden;

FIG. 5 zeigt gruppierte Fixationen im Blickfeld eines Benutzers an einem Beispiel;

FIG. 6A zeigt kumulierte Fixationshäufigkeiten an einem Beispiel;

FIG. 6B zeigt kumulierte Fixationsdauern an einem Beispiel;

**Detailbeschreibung**

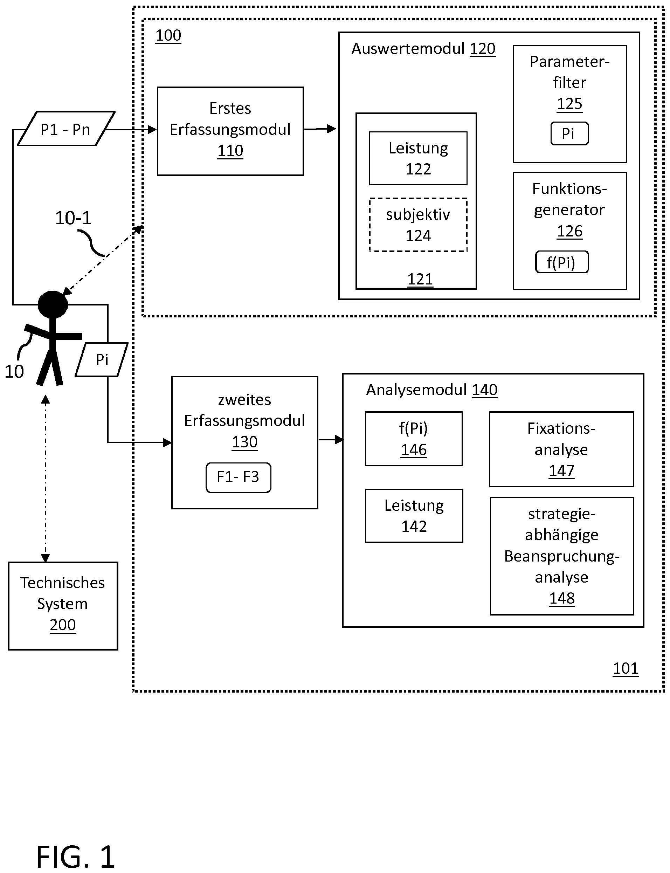

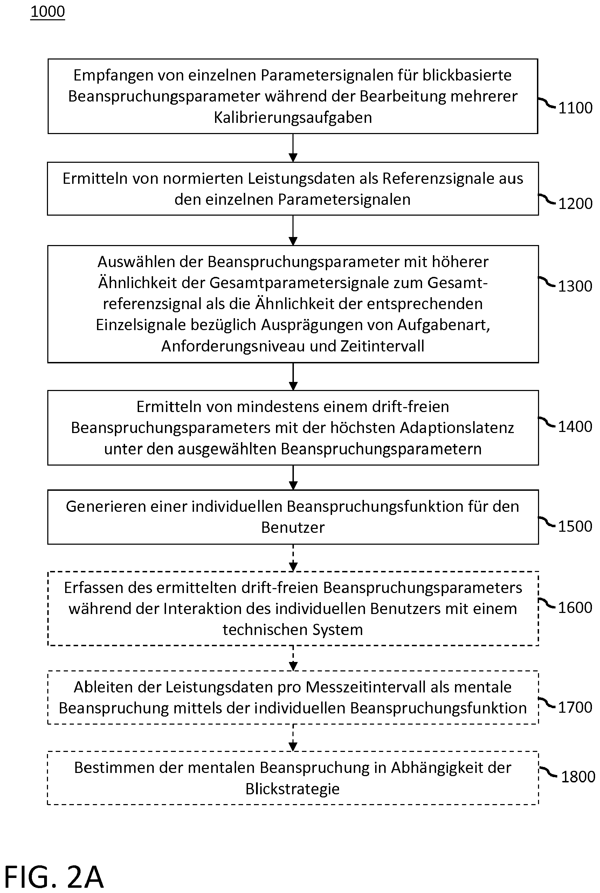

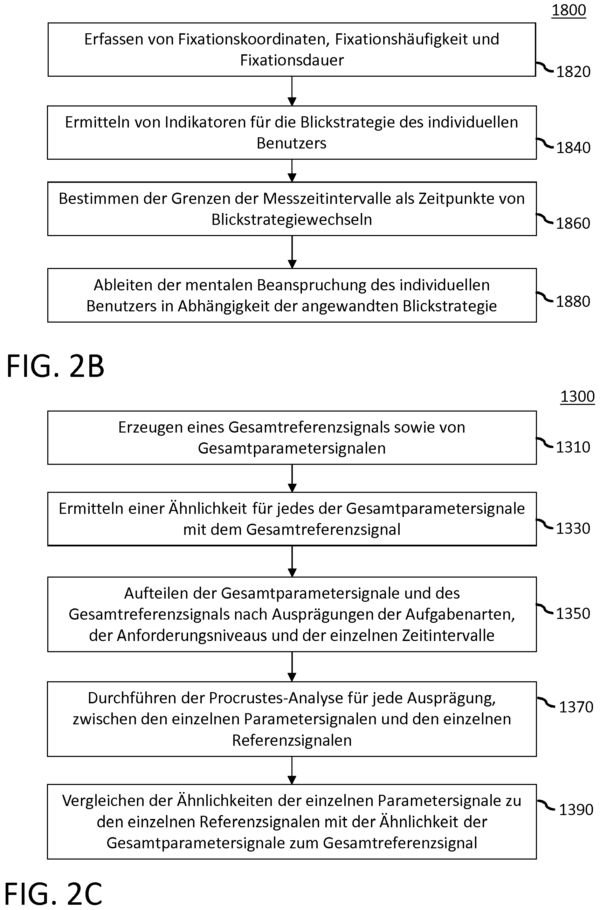

FIG. 1 zeigt ein Ausführungsbeispiel 100 eines Computersystems zur Bestimmung eines geeigneten Verfahrens für die quantitative Messung der mentalen Beanspruchung eines individuellen Benutzers 10 mit einem technischen System 200, sowie außerdem ein erweitertes Ausführungsbeispiel 101 zur Durchführung des Verfahrens. FIG. 2A zeigt ein Flussdiagramm 1000 zur Veranschaulichung der erfindungsgemäßen Verfahren. Die FIG. 2B - 2D zeigen weitere Flussdiagramme zur detaillierteren Darstellung einzelner Verfahrensschritte von FIG. 2A. Die folgende Beschreibung der FIG. 1 erläutert die Funktionen des Computersystems 100/101 im Lichte der Verfahrensschritte aus den Figuren 2A bis 2D. Die folgende Beschreibung verwendet daher die Bezugsziffern der FIG. 1 und der Figuren 2A - 2D.

Die mentale Beanspruchung beschreibt die kognitive Reaktion des menschlichen Informationsverarbeitungssystems auf äußere Belastungen in Abhängigkeit von seinen persönlichen Vorrausetzungen und den individuellen Bewältigungsstrategien (Packebusch 2003; Niederl 2007). Die mentale Beanspruchung kann über physiologische Reaktionen des Menschen (Okulometrie, EKG, EDA), leistungsbasierte Parameter (Fehler in Haupt- und Nebenaufgaben) und das subjektive Empfinden (Fragebögen) erfasst werden (Manzey 1998). Vor dem Hintergrund der praktischen Anwendbarkeit außerhalb des Labors müssen jedoch einige Einschränkungen der Erhebungsmethoden berücksichtigt werden. Die leistungsbasierten Parameter, wie die Fehlerquote in einer Nebenaufgabe, zählen zu den zuverlässigsten Indikatoren für die mentale Beanspruchung (Wickens, C. D. et al. (2013). Engineering psychology and human performance (Fourth edition). Boston: Pearson). Das subjektive Empfinden 124 ist ebenfalls ein guter Indikator, weicht jedoch häufig von den Leistungsdaten ab, da sich Personen unterschiedlich stark bei der Bearbeitung einer Aufgabe anstrengen können oder sich selbst einfach anders einschätzen (Wickens et al. 2013). Weiterhin kann aus der Kombination von leistungsbasierten Parametern und subjektivem Empfinden die mentale Effizienz als Anstrengung/Leistung abgeleitet werden, die ebenfalls als Indikator für die mentale Beanspruchung herangezogen werden kann. Physiologische Parameter nutzen Reaktionen des menschlichen Körpers, um kontinuierlich und in hoher zeitlicher Auflösung auf die mentale Beanspruchung zu schließen (Manzey 1998).

Das Computersystem 100 zur Bestimmung eines geeigneten Messverfahrens ist über eine geeignete Schnittstelle mit einem Blickerfassungssystem (nicht dargestellt) gekoppelt, welches blickbasierte Beanspruchungsparametersignale P1 - Pn des Benutzers 10 erfasst. Dabei kann es sich um ein handelsübliches kopfbasiertes Blickerfassungssystem handeln oder um ein fest installiertes Blickerfassungssystem, welches über eine oder mehrere Kameras das Blickverhalten des individuellen Benutzers 10 erfasst (z.B. Kamera(s) zur Überwachung eines Fahrzeugführers während der Fahrt). Blickerfassungssysteme bieten eine hohe Nutzerakzeptanz bei gleichzeitiger geringer Latenz zu der menschlichen Informationsverarbeitung und eine hohe Sensitivität (Sträter 2005; Manzey 1998). Gleichzeitig kann das Blickerfassungssystem zur Erfassung der Blickstrategie (Fixationskoordinaten, - häufigkeiten, -dauern) des Benutzers genutzt werden. Der erfindungsgemäße individuelle Ansatz der Erfindung ist durch eine sehr hohe Genauigkeit geprägt. Im Stand der Technik wurde bisher ein individueller Ansatz aufgrund von Problemen der Vergleichbarkeit zwischen Personen und des damit verbundenen Aufwands als nicht praktikabel erachtet.

Das System 100 umfasst ein erstes Erfassungsmodul 110 welches die einzelnen Parametersignale P1 - Pn für mehrere blickbasierte Beanspruchungsparameter des individuellen Benutzers 10 empfängt 1100, während dieser dabei mehrere Kalibrierungsaufgaben über jeweils zwei oder mehr Kalibrierungszeitintervalle hinweg bearbeitet. Die Kalibrierungsaufgaben umfassen dabei zwei oder mehr verschiedene Aufgabenarten mit jeweils zwei oder mehr verschiedenen Anforderungsniveaus umfassen. Die Kalibrierungsaufgaben können über ein optionales Aufgabenmodul des Systems gestellt werden, mit dem der Benutzer über eine entsprechende Benutzerschnittstelle 10-1 interagiert. Alternativ ist es auch möglich, die Kalibrierungsaufgaben über ein separates System zu bearbeiten, welches die Leistungsdaten für die einzelnen Kalibrierungsaufgaben dann an das System 100 übermittelt.

Ein Auswertemodul 120 des Systems 100 ermittelt 1200 normierte Leistungsdaten 122 als einzelne Referenzsignale 121 für die jeweiligen Aufgaben aus den einzelnen Parametersignalen P1 - Pn. Die Leistungsdaten stellen dabei ein Maß für die mentale Beanspruchung des Benutzers während der Bearbeitung der Kalibrierungsaufgaben dar. In anderen Worten, die Leistungsdaten beschreiben die Güte der Lösung einzelner Kalibrierungsaufgaben durch den Benutzer 10.

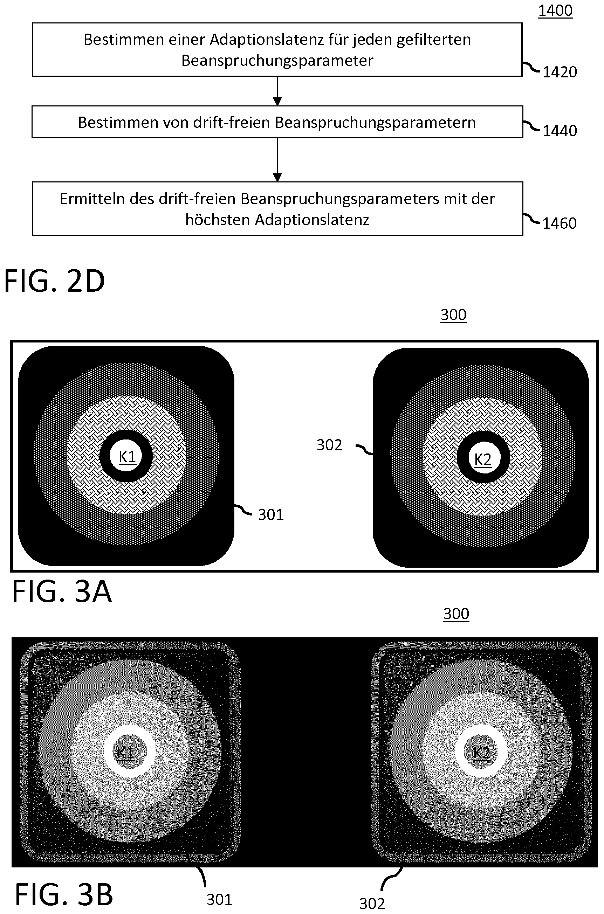

FIG. 3A, 3B zeigt ein Beispiel für eine Kalibrierungsaufgabe 300, die zur Bestimmung des geeigneten Messverfahrens eingesetzt werden kann. FIG. 3A zeigt die Kalibrierungsaufgabe 300 in einer Schwarz-Weiß-Darstellung. FIG. 3B zeigt die selbe Kalibrierungsaufgabe 300 in einer Graustufen-Darstellung, die eine realistischeres Bild des tatsächlichen Erscheinungsbilds vermittelt. Beim Bearbeiten der Kalibrierungsaufgabe werden Datensätze generiert, aus denen ein individuelles Beanspruchungsmodell (Beanspruchungsfunktion) für den Benutzer 10 abgeleitet werden kann. Für die Kalibrierungsaufgabe wird der Benutzer 10 instruiert, sodass alle folgenden Aufgaben und die Messinstrumente (physiologisch, leistungsbasiert, subjektiv) bearbeitet werden können. Die Kalibrierungsaufgabe kann z.B. auf einem Kalibrierungsclient (z.B., Tablet-PC, Laptop, oder Client Device des Systems 100) bearbeitet werden und umfasst mehrere Teilaufgaben, die jeweils aus einer Haupt- und einer Nebenaufgabe bestehen. Im Beispiel der FIG. 3 sind die Hauptaufgaben aus dem Bereich der Flugführung abgeleitet und bestehen aus zwei nebeneinander angeordneten zweidimensionalen Feldern 301, 302, die verschiedene Ringe unterschiedlicher Farbgebung umfassen. In der Mitte der Felder 301, 302 befindet sich jeweils eine gefärbte "Kugel" (dargestellt als Kreisflächen K1, K2), die unabhängig voneinander über eine Tastatur oder eine Steuerungseinheit des Kalibrierungsclients steuerbar sind. Die Kugeln K1, K2 bewegen sich während der Aufgaben unterschiedlich stark in zufällige Richtungen aus der Mitte heraus. Die Aufgabe für den Benutzer besteht darin, die Kugeln so präzise wie möglich über Gegensteuerungsmaßnahmen in der Mitte zu halten (z.B., mittels eines Joysticks oder ähnlicher Kontrollmittel) oder, falls eine Kugel die Mitte verlassen sollte, diese so schnell und so genau wie möglich in die Mitte zurück zu steuern.

Im Beispiel besteht die Hauptaufgabe aus zwei Abschnitten. Im ersten Teil (Hauptaufgabe 1) bewegt sich ausschließlich die linke Kugel K1, wohingegen sich im zweiten Teil (Hauptaufgabe 2) beide Kugeln K1, K2 bewegen und dementsprechend auch beide Kugeln gesteuert werden müssen. Beide Aufgabenteile werden mindestens zweimal durchgeführt, wobei sie jeweils mit einer von mindestens zwei möglichen Nebenaufgaben kombiniert werden.

Eine Nebenaufgabe kann z.B. eine arithmetische Nebenaufgabe sein, die aus Subtraktionsaufgaben im Zahlenraum von 1-100 besteht, welche dem Benutzer in einem vorgegeben Sekundentakt (z.B. alle 3, 4, 5, etc. Sekunden) über einen Audiokanal (z.B. Lautsprecher oder Kopfhörer) präsentiert werden. Ggf. können auch Additions-, Multiplikations- oder Divisionsaufgaben verwendet werden. Der Benutzer soll so schnell wie möglich mit dem möglichst richtigen Ergebnis verbal antworten. Die Antwort wird über ein Mikrophon aufgezeichnet. Die Priorität soll dabei aber immer auf der Hauptaufgabe liegen.

Eine weitere Nebenaufgabe kann z.B. eine visuelle Nebenaufgabe sein, die aus einem visuellen Reiz besteht. Beispielsweise kann dieser Reiz in Form eines $0,5 \times 0,5mm^2$ großen weißen Quadrats bestehen, welches in unterschiedlichen zeitlichen Abständen und in zufälligen Ecken des Bildschirms für jeweils eine kurze Dauer von weniger als einer Sekunde (z.B. 0,3 Sekunden) angezeigt wird. Der Benutzer soll so schnell wie möglich mit einer Eingabe (z.B. einem Druck auf eine Taste) bestätigen, dass der Punkt gesehen oder wahrgenommen wurde. Auch in dieser Nebenaufgabenbedingung soll die Priorität der Aufgabenbearbeitung auf der Hauptaufgabe liegen.

Im obigen Beispiel ergeben sich somit insgesamt vier Anforderungsniveaus (s. Tabelle 1), wobei die Reihenfolge der visuellen und der arithmetischen Nebenaufgabenbedingungen für verschiedene Benutzer randomisiert werden kann, um Reihenfolgeeffekte zu vermeiden. Jedes Anforderungsniveau wird durch den Benutzer während eines Kalibrierungszeitintervalls bearbeitet, welches eine Zeitdauer aufweist, die die im Vergleich zur Taktung der Nebenausgaben lang genug ist, um ausreichend viele Nebenaufgaben zu bearbeiten. Im dargestellten Beispiel hat sich eine Dauer von 1 Minute für die Kalibrierungszeitintervalle als angemessen erwiesen.

Tabelle 1 - Anforderungsniveaus der Kalibrierungsaufgabe

| Nebenaufgabe | Hauptaufgabe |
|---|---|
| | |
| arithmetische Nebenaufgabe | Hauptaufgabe 1 (eine Kugel) |
| | Hauptaufgabe 2 (zwei Kugeln) |
| visuelle Nebenaufgabe | Hauptaufgabe 1 (eine Kugel) |
| | Hauptaufgabe 2 (zwei Kugeln) |

Für die Durchführung der Kalibrierungsaufgaben wird zunächst das Blickerfassungssystem für den Benutzer justiert und technisch kalibriert (ggf. Anpassung an die individuelle Kopfform des Benutzers und Synchronisierung der Kameras), was die Ausrichtung der Kameras und die Überprüfung der korrekten Augenerkennung beinhaltet. Sobald das Eye-Tracking System erfolgreich kalibriert wurde, wird die Reihenfolge der (z.B. vier) Anforderungsniveaus für den Benutzer festgelegt. Nachfolgend wird die Aufzeichnung der Blickerfassung über eine dazugehörige Software gestartet und der Benutzer beginnt mit der Bearbeitung der verschiedenen Anforderungsniveaus (z.B. $x \in [1, 4]$). Nach der Bearbeitung der Aufgabe am Ende eines Kalibrierungszeitintervalls wird die Aufzeichnung beendet und die Journal-Dateien als Ausgabe des Blickerfassungssystems sowie die Leistungsdaten gesichert. Journal-Dateien können beispielsweise im CSV-Format exportiert werden und beinhalten Augenpositionen (X- und Y-Koordinaten), Fixationskoordinaten (X und Y) und Pupillenflächen, die über die Zeit Frame für Frame aufgezeichnet werden. Die Leistungsdaten umfassen die Fehlerquoten der Nebenaufgaben. Bei der visuellen Nebenaufgabe können zusätzlich zu den Fehlern auch die Reaktionszeiten erfasst werden. Optional kann während der Sicherung der Daten das subjektive Beanspruchungsempfinden des Probanden mittels eines Fragebogens (NASA-TLX oder RSME) erfasst werden und im Anschluss ebenfalls gesichert werden. Diese Vorgänge werden für alle Anforderungsniveaus wiederholt und im Anschluss werden die Ausgaben der Kalibrierungsaufgaben an das Auswertemodul 120 übermittelt. Die Leistungsdaten, bestehend aus Fehlern und Reaktionszeiten, werden normiert (normierte Leistungsdaten 122). Für die arithmetische Nebenaufgabe wird z.B. die Fehlerquote berechnet und für die visuelle Nebenaufgabe kann für den Leistungswert $NA_{Vis}$ die Fehlerquote zusätzlich mit den Reaktionszeiten kombiniert werden:

$$Leistungswert\ NA_{Vis} = 0,5 \times \frac{Fehler}{Anzahl\ Reize} + 0,5 \times \frac{mittlere\ Reaktionszeit - 0,3}{1,1}$$

Dadurch wird ein vergleichbarer und normierter Wert erhalten, ohne Informationen über das Benutzerverhalten zu verlieren.

In einer Ausführungsform werden auch die subjektiven Angaben der Fragenbögen normiert, indem die angegeben Werte durch die Skalenlänge dividiert werden. Am Ende findet nochmals eine ganzheitliche Prüfung der Daten auf mögliche Fehler, wie fehlende oder falsche Werte statt, bevor dann aus den Journal-Dateien insgesamt sieben okulare Beanspruchungsindikatoren in unterschiedlichen Varianten (z. B. verschiedene Filter) nach standardisierten Vorgaben automatisiert berechnet werden können.

Tabelle 2 enthält eine Übersicht über diese Parameter und deren Berechnung. Nach der Berechnung werden die Parameter gemäß Theorien der menschlichen Informationsverarbeitung (Dehaene und Changeux 2005; Kiefer et al. 2012) in bewusste (kBew = 23) und unbewusste (kUnbew = 38) Indikatoren eingeteilt. Bewusste Indikatoren können zum einen während der Aufgabe durch den Benutzer reproduzierbar beeinflusst werden und zum anderen repräsentieren sie verstärkt die bewusst ablaufenden Informationsverarbeitungsprozesse (Denkprozesse des Benutzers).

Tabelle 2 - blickbasierte Beanspruchungsparameter

| Parameter | Kurzbeschreibung | Indikator für... |
|---|---|---|
| *Fixationsdauer* | Durchschnittliche Dauer von Fixationen (für z. B. 2 Varianten - Variation bezüglich des Betrachtungsi nterva lls) | |
| *Sakkadenlänge* | Durchschnittliche Länge von Blickpfaden (für z. B. 2 Varianten -Variation bezüglich des Betrachtungsintervalls) | bewusste (beeinflussbare) Verarbeitungsprozesse |
| *Nearest Neighbor Index (NNI)* | Verteilung von Fixationen im Blickfeld (für z. B. 19 Varianten - Variation bezüglich der Referenzfläche) | |
| *Blinzelrate* | Anzahl des Blinzelns in definierten Zeitintervallen (für z. B. 2 Varianten - Variation bezüglich des Betrachtungsintervalls) | |
| *Blinzeldauer* | Mittlere Dauer des Blinzeln (für z. B. 2 Varianten - Variation bezüglich des Betrachtungsintervalls) | unbewusste (nichtbeeinflussbare) Verarbeitungsprozesse |
| *PERCLOS* | Kumulierte Dauer des Blinzelns (für z. B. 2 Varianten - Variation bezüglich des Betrachtungsintervalls) | |
| *Pupillendurchschnitts-variabilität* | Mittlere Anzahl an Fluktuationen der Pupille (für z. B. 32 Varianten -Variation bezüglich des Signalfilters) | |

Unbewusste Indikatoren können nicht beeinflusst werden und indizieren überwiegend unbewusst ablaufende Verarbeitungsprozesse. Diese Einteilung ermöglicht eine höhere Genauigkeit bei der Bestimmung des individuellen Messverfahrens, die mit bisherigen Stand der Technik nicht erreicht wird. Die Vielzahl an Varianten der Parameter ist dabei vorteilhaft, um die individuell stark unterschiedlichen Reaktionen einzelner Benutzer vollständig abbilden zu können

Die nun folgenden Schritte werden durch den Parameterfilter 125 (s. FIG. 1) des Auswertemoduls 120 ausgeführt und dienen der Reduktion der Beanspruchungsparametersignale P1 - Pn auf möglichst ein für den Benutzer 10 charakteristisches Parametersignal Pi. Es können allerdings auch mehr als ein Parametersignal als Ergebnis der Filterung bestimmt werden.

In einem ersten Filterungsschritt wählt 1300 der Parameterfilter 125 solche Beanspruchungsparameter aus, deren Gesamtparametersignale eine höhere Ähnlichkeit zum Gesamtreferenzsignal aufweisen als die Ähnlichkeit ihrer einzelnen Parametersignale zu den jeweiligen einzelnen Referenzsignalen für die einzelnen Ausprägungen von Aufgabenart, Anforderungsniveau und Zeitintervall. FIG. 2B zeigt dabei Details des Auswählens 1300 mittels einer Procrustes-Analyse. Die Funktion der Procrustes-Analyse ist in (Osis, S. T. et al. (2015). A novel method tp evaluate error in anatomical marker placement using a modified generalized Procrustes analysis. Computer methods in biomechanics and biomedical engineering (10), S. 1108 - 1116) im Detail beschrieben. Dabei werden zunächst das Gesamtreferenzsignal aus den einzelnen Referenzsignalen sowie die Gesamtparametersignale aus den einzelnen Parametersignalen für jeden erfassten Beanspruchungsparameter durch Aneinanderreihen der jeweiligen Messergebnisse für alle Teilaufgaben der Kalibrierungsaufgaben erzeugt 1310. Im Anschluss ermittelt 1330 der Parameterfilter 125 eine Ähnlichkeit für jedes der Gesamtparametersignale mit dem Gesamtreferenzsignal über die Procrustes-Analyse. Die Gesamtparametersignale und das Gesamtreferenzsignals werden dann nach den Ausprägungen der Aufgabenarten, der Anforderungsniveaus und der einzelnen Zeitintervalle aufgeteilt 1350. Nun wird die Procrustes-Analyse für jede Ausprägung, zwischen den einzelnen Parametersignalen und den einzelnen Referenzsignalen, durchgeführt 1370. Schließlich werden die Ähnlichkeiten der einzelnen Parametersignale zu den einzelnen Referenzsignalen mit der Ähnlichkeit der Gesamtparametersignale zum Gesamtreferenzsignal verglichen 1390.

Die oben beschriebene Auswahlschritt 1300 mittels einer Procrustes Analyse kann als erster Schritt der Modellbildung zur Ableitung einer individuellen Beanspruchungsfunktion gesehen werden. Bei der Procrustes-Analyse handelt es sich um ein Verfahren aus dem Bereich der Mustererkennung, welches überwiegend in der Bildverarbeitung oder der Biomechanik Anwendung findet (Osis, S. T. et al. (2015), S. 1108 - 1116) . Das Computersystem 100 verwendet dieses Verfahren in der Analyse von blickbasierten Beanspruchungsparametern. Die Procrustes-Analyse ist ein dreistufiges Verfahren zur Ermittlung wie ähnlich sich zwei Signale sind. Zunächst werden das Referenzsignal und Signal des zu untersuchenden okularen Parameters in m x 2 Matrizen (m = Anzahl der Messpunkte) aufbereitet, bevor beide dann translatiert werden (Subtraktion des Mittelwertes), sodass der Mittelpunkt beider Matrizen im Ursprung liegt. Im Anschluss wird die Größe beider Matrizen vereinheitlicht, indem die einzelnen Werte durch einen jeweiligen Skalierungsfaktor s dividiert werden (Osis et al. 2015, S. 1110).

$$s = \sqrt{\frac{\sum_{n=1}^{k}(x_n^2 + y_n^2)}{k}}$$

Jetzt wird die Matrize des Parameters um $\alpha = 1°$, mit $\alpha \in [1, 360]$, nach rechts rotiert. Im Anschluss wird die mittlere quadratische Abweichung d zu der Referenzmatrix berechnet. Dieser Vorgang wiederholt sich, bis zu jedem $\alpha$ des Wertebereichs eine Abweichung der Signale vorliegt. Im Anschluss wird der Winkel $\alpha$ ausgewählt, für den gilt d = min{d}. Die Werte für d und $\alpha$ werden für den jeweiligen Parameter gespeichert. Sie beschreiben, wie weit das Parametersignal an das Referenzsignal unter welcher Veränderung angenähert werden kann, oder in anderen Worten, wie ähnlich sich beide Signale sind. Diese Analyse wird für jeden Parameter k in verschiedenen Varianten durchgeführt, um die essentiellen Einflussfaktoren auf die blickbasierten Parameter erstmals quantifizieren zu können (s. Tabelle 3).

Tabelle 3 - Aufteilung der Signale für die Procrustes-Analyse

| Eingangssignal | Matrix | Verwendung |
|---|---|---|
| *Gesamte Kalibrierung* (4 x 2 Datenpunkte) | 8 x 2 | Quantifizierung der Ähnlichkeit des Gesamtsignals |
| *Leicht* (Hauptaufgabe 1; 2 x 2 Datenpunkte | 4 x 2 | Aufteilung des Signals nach Anforderungsniveau (z.B. leicht / schwer) |
| *Schwer* (Hauptaufgabe 2; 2 x 2 Datenpunkte | 4 x 2 | |
| *Visuell* (Nebenaufgabe visuell; 2 x 2 Datenpunkte) | 4 x 2 | Aufteilung der Signals nach Aufgabenart (z.B. arithmetisch / visuell) |
| *Visuell* (Nebenaufgabe visuell; 2 x 2 Datenpunkte) | 4 x 2 | |
| *Aufgabenbeginn* (0-30s; 2 x 2 Datenpunkte | 4 x 2 | Aufteilung der Signals nach der Aufgabendauer (z.B. 0-30s / 30-60s) |
| *Aufgabenende* (30-60s; 2 x 2 Datenpunkte | 4 x 2 | |

Im Anschluss werden die mittleren Abweichungen für die einzelnen Signalvarianten verglichen und nur Parameter weiter berücksichtigt, die die folgende Bedingung erfüllen:

$d(gesamt) < d_{MW}(Anforderungsniveaus)$ &&

$$d(gesamt) < d_{MW}(Aufgabenarten) \text{ \&\&}$$

$$d(gesamt) < d_{MW}(Aufgabendauern)$$

Wenn diese drei Bedingungen erfüllt sind, kann davon ausgegangen werden, das der jeweilige Parameter anforderungs-, aufgabenart- und aufgabendauerunabhängig ist, was eine Voraussetzung für eine hoch zuverlässige Beanspruchungsmessung in Unabhängigkeit von dem zu betrachtenden technischen System ist.

Der nächste Schritt, ermitteln 1400 (s. FIG. 2A) von mindestens einem drift-freien Beanspruchungsparameter (Pi) mit der höchsten Adaptionslatenz unter den ausgewählten Beanspruchungsparametern, beinhaltet tatsächlich zwei weitere Filterungsschritte, wie in FIG. 2D dargestellt. Zunächst wird für jeden (im Auswahlschritt 1300) gefilterten Beanspruchungsparameter einer Adaptionslatenz mittels einer Moving-Correlation-Analyse bestimmt 1420 und im Anschluss die mittleren Adaptionslatenz von bewussten Beanspruchungsparametern mit der mittleren Adaptionslatenz von unbewussten Beanspruchungsparametern verglichen. Schließlich werden die Beanspruchungsparameter der Kategorie mit der höheren mittleren Adaptionslatenz selektiert. Aus den selektierten Beanspruchungsparametern werden nun drift-freie Parameter bestimmt 1440, die im Betrachtungsbereich des Referenzsignals nicht von dem Referenzsignal abdriften. Schließlich wird der drift-freie Beanspruchungsparameters mit der höchsten Adaptionslatenz ermittelt 1460.

Mit anderen Worten, in einer Ausführungsform werden für die verbleibenden (ausgewählten) Parameter jeweils drei Korrelationen mit der Referenzgröße gebildet. Neben der normalen Korrelation werden auch zwei Korrelationen basierend auf einer Verschiebung der Parametersignals berechnet. Das Parametersignal wird einmal um einem Messzeitpunkt nach vorne verschoben (Korrelation-Plus) und einmal nach hinten (Korrelation-Minus). So kann festgestellt werden, ob sich ein Parameter schon vor einer Änderung der Referenzgröße verändert (proaktive Adaption) oder erst danach (reaktive Adaption). Dieses Vorgehen erlaubt, die Adaptionslatenz einzelner Parameter in der Vorhersage zu berücksichtigen.

Im nächsten Schritt wird geprüft ob die normale Korrelation eines Parameters besonders hoch ist (z.B., Korrel(normal) > OG95%(Bew / Unbew)) und der Winkel a, um den das Signal in der Procrustes-Analyse rotiert wurde, besonders klein ist (z.B., α(gesamt) < UG95%(Bew / Unbew)). Dazu werden beispielsweise die 95%-Konfidenzintervalle der einzelnen Betrachtungsgrößen über die verbleibenden bewussten und unbewussten Parameter berechnet. Andere Schwellwerte für die Konfidenzintervalle können unter Berücksichtigung der Rechenzeit und gewünschten Genauigkeit ebenfalls gewählt werden. Die verbleibenden bewussten und unbewussten Parameter werden über diesen Schritt noch weiter reduziert. Folgend werden die mittleren Adaptionseffekte der bewussten Parameter mit denen der unbewussten Parameter verglichen. Die mittleren Adaptionseffekte (AE) können dabei nach dem folgenden Muster bestimmt werden:

$$Wenn\ Max(korrel) = Normal,\ dann\ AE = 0$$

$$Wenn\ Max(korrel) = Plus,\ dann\ AE = |\ Korrel(Plus) - Korrel(normal)\ |$$

$$Wenn\ Max(korrel) = Minus,\ dann\ AE = |\ Korrel(Minus) - Korrel(normal)\ |$$

Ein hoher mittlerer Adaptionseffekt spricht dabei für ein einheitliches Verhalten bewusster oder unbewusster Parameter, sodass im Folgenden nur noch Parameter der Kategorie berücksichtigt werden, die einen höheren mittleren Adaptionseffekt aufweisen. Als letztes Kriterium wird dann anhand der Steigungswinkel φ der Ursprungssignale überprüft, ob der jeweilige Parameter und die Referenzgröße auseinander driften. Nur wenn die folgende Kausalitätskette erfüllt ist, kann sichergestellt werden, dass die Parameter sich bei besonders hohen Werten der Referenzgröße ebenfalls ähnlich verhalten wie in den betrachteten Messpunkten:

$$Wenn\ \phi\ (Parameter) > \phi\ (Referenz)\ \&\&\ Min(Parameter) < Min(Referenz),\ dann\ kein\ Drift$$

$$Wenn\ \phi\ (Parameter) < \phi\ (Referenz)\ \&\&\ Min(Parameter) > Min(Referenz),\ dann\ kein\ Drift$$
$$sonst,\ Drift\ (Ausschluss)$$

Sollten jetzt nach Anwenden der Filterschritte noch mehrere Parameter vorhanden sein, die die genannten Anforderungen erfüllen, so wird der Parameter P(i) mit dem deutlichsten Adaptionseffekt ausgewählt, da dieser die eindeutigste Vorhersage ermöglicht. Sollte kein Parameter die Anforderungen erfüllen, ist für diesen Probanden keine hoch zuverlässige Vorhersage möglich und es wird der Parameter ausgewählt, der die meisten Anforderungen erfüllt. Dies wird vorzugsweise dem Benutzer rückgemeldet.

Nachdem der Beanspruchungsparameter P(i) ermittelt wurde, der die eindeutigste Vorhersage ermöglicht, kann nun eine individuelle Beanspruchungsfunktion f(Pi) für den Benutzer 10 generiert 1500 werden. Dies erfolgt durch Überführen von mindestens einem der ermittelten drift-freien Beanspruchungsparameter Pi in eine individuelle lineare Funktion $F_{Individuell}$ mittels linearer Regression aus den während der Kalibrierungsaufgabe für den mindestens einen der ermittelten drift-freien Beanspruchungsparameter Pi erfassten Leistungsdaten 122.

$$F_{Individuell} = b_0 + b_1 \times P$$

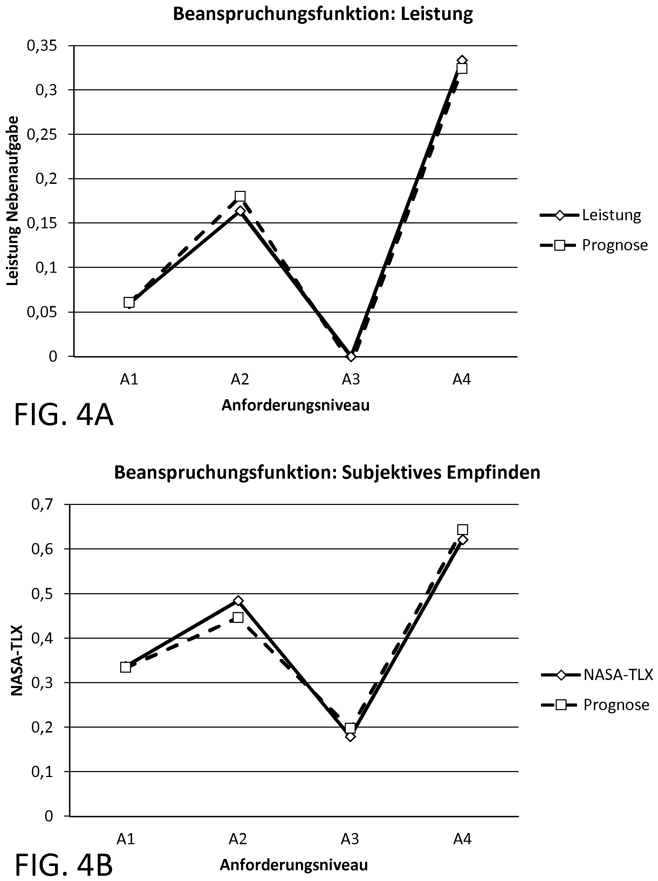

Die Konstanten b0 und b1 werden über eine lineare Regression aus der Vorhersage der jeweiligen Parameter der verschiedenen (z.B. vier) Anforderungsniveaus (z.B. 8 Messpunkte pro Kalibrierungsaufgabe) berechnet. Durch die Konstanten wird sowohl die Varianzaufklärung, als auch die mittlere Abweichung der Vorhersage von den tatsächlichen Werten optimiert. Durch diese individuelle Funktionen kann eine mittlere Varianzaufklärung von r2 = 0,976 (SD = 0,165) erreicht werden, was die Stand der Technik erreichten Varianzaufklärungen um ein Vielfaches übersteigt. Die Figuren 4A, 4B verdeutlichen grafisch die hohe Varianzaufklärung und die geringe mittlere Abweichung der Regression von den tatsächlichen Datenpunkten aus vier beispielhaften Anforderungsniveaus der Kalibrierungsaufgabe. Dabei zeigt FIG. 4A die Beanspruchungsfunktion für das Referenzsignal Leistung und FIG. 4B für das Referenzsignal subjektives Empfinden.

Das Computersystem 100 (s. FIG. 1) kann als Kalibrierungsmodul für das erfindungsgemäße Computersystem 101 zur quantitativen Messung der mentalen Beanspruchung des individuellen Benutzers bei dessen Interaktion mit dem technischen System 200 gesehen werden. Mittels des Kalibrierungsmoduls 100 kann innerhalb weniger Minuten das für den Benutzer 10 optimierte Messverfahren bestimmt werden, welches das zu messende Parametersignal aus einer Vielzahl (ggf. mehr als 60 mögliche Parametersignale) auswählt und die dazugehörige Beanspruchungsfunktion generiert. Das auf diese Weise bestimmte und kalibrierte Messverfahren kann nun eingesetzt werden, um mit dem erweiterten Computersystem 101 die tatsächliche mentale Beanspruchung des Benutzers während seiner Interaktion mit dem technischen System 200 (oder einem belieben anderen technischen System) zu messen. Das Kalibrierungsmodul 100 kann aber auch als ein separates System implementiert werden, welches lediglich mit den zusätzlichen Komponenten 130, 140 des Computersystems 101 kommunikativ gekoppelt ist (z.B. über ein Netzwerk).

Das Computersystem 100 umfasst für die Beanspruchungsmessung ein zweites Erfassungsmodul 130 zum Erfassen 1600 des mindestens einen ermittelten drift-freien Beanspruchungsparameters Pi während der Interaktion des individuellen Benutzers mit dem technischen System 200 über mehrere Messzeitintervalle. Zu diesem Zweck wird das identische oder ein baugleiches Blickerfassungssystem verwendet, welches für die Kalibrierung eingesetzt wurde.

Ein Analysemodul 140 des Computersystems 101 leitet 1700 dann die Leistungsdaten 142 pro Messzeitintervall als mentale Beanspruchung mittels der individuellen Beanspruchungsfunktion fP(i) ab. Die Beanspruchungsfunktion fP(i) wird vom Kalibrierungsmodul 100 nach ihrer Generierung für das Analysemodul 140 bereit gestellt. Dies kann entweder in Form eines Dienstes erfolgen, bei dem fP(i) als ein "remote service" bereitgestellt wird oder aber das Auswertemodul 140 kann selbst die Funktion in einem entsprechenden Berechnungsmodul 146 speichern.

In einem Ausführungsbeispiel handelt es sich bei dem technischen System 200 um ein Navigationssystem eines Fahrzeugs. Das System 101 kann nun die mentale Beanspruchung des Fahrers (Benutzer 10) während einer Autofahrt untersuchen und dabei z.B. für ein neu entwickeltes Navigationsdisplay die Ablenkungswirkung des Displaylayouts messen.

Dazu wird der Fahrer mit dem Blickerfassungssystem ausgerüstet. Allerdings werden nun während der Fahrt keine leistungsbasierten oder subjektiven Daten erfasst, da diese aus dem Beanspruchungsmodell fP(i) abgeleitet 1700 werden können. Nachdem der Fahrer die Aufgabe bearbeitet oder durchgeführt hat, wird die Journal-Datei des Blickerfassungssystems gesichert, geprüft und bereinigt. Bevor die detaillierte Analyse der Beanspruchung vorgenommen werden kann, werden die zeitlichen Betrachtungsintervalle festgelegt. Beispielsweise können standardmäßig Messzeitintervalle von t = 30s verwendet werden, da diese eine valide Berechnung der einzelnen Parameter ermöglichen. Bei einer Fahrtätigkeit von fünf Minuten können demnach zehn Intervalle gebildet werden, für die jeweils die Beanspruchungsfunktion basierend auf dem ausgewählten Parameter zur Bestimmung der Referenzinformation verwendet wird.

30 Sekunden Messzeitintervalle berücksichtigen jedoch nicht das individuelle Verhalten der Person oder den Verlauf der Aufgabe. Um die Strategien der Probanden zu berücksichtigen und die Grenzen der Zeitintervalle auf Zeitpunkte von Strategiewechseln zulegen wird eine Clusteranalyse des Blickverhaltens durchgeführt. Zu diesem Zweck kann das Auswertemodul 140 weiterhin ein Fixationsanalysemodul 147 und ein Modul 148 für die strategieabhängige Beanspruchungsanalyse beinhalten. Weiterhin kann das zweite Erfassungsmodul 130 in diesem Ausführungsbeispiel auch Fixationskoordinaten F1, Fixationshäufigkeit F2 und Fixationsdauer F3 des individuellen Benutzers 10 während der Interaktion mit dem technischen System 200 erfassen 1820, wie in FIG. 2B dargestellt.

Das Fixationsanalysemodul 147 ermittelt 1840 nun Indikatoren für die Blickstrategie des individuellen Benutzers mit einer Kombination aus einer Clusteranalyse der Fixationskoordinaten F1 und einer Analyse der Fixationshäufigkeit F2 und Fixationsdauer F3. Das strategieabhängige Modul 148 bestimmt 1860 die Grenzen der Messzeitintervalle als Zeitpunkte von Blickstrategiewechseln mittels Veränderungen in den Ergebnissen der Clusteranalyse der Fixationskoordinaten oder der Analyse von Fixationshäufigkeit und -dauer und leitet 1880 die mentale Beanspruchung des individuellen Benutzers in Abhängigkeit der angewandten Blickstrategie ab.

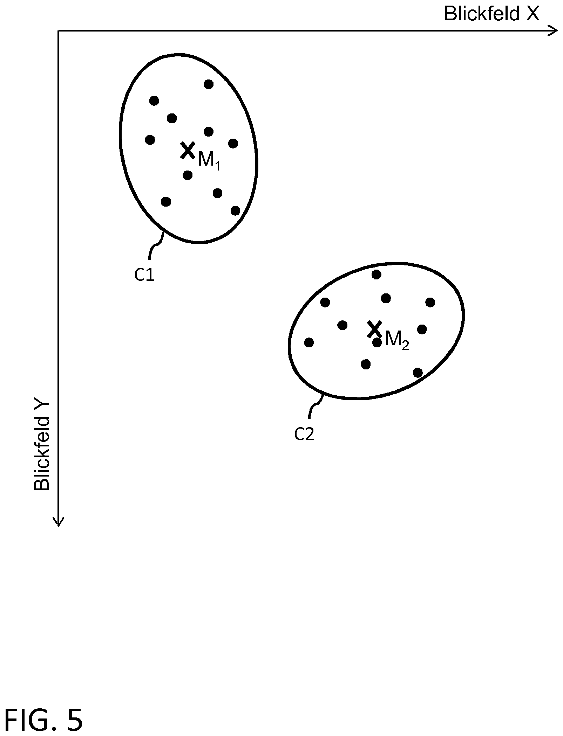

So ist es möglich, die mentale Beanspruchung in Abhängigkeit der Blickstrategie der Person zu bestimmen 1800 und so direkte Gestaltungsempfehlungen für das technische System 200 abzuleiten. FIG. 5 zeigt gruppierte Fixationen im Blickfeld des Benutzers. Eine Fixation ist dabei als schwarzer Punkt dargestellt. Für die Clusteranalyse werden Fixationskoordinaten (X und Y) (z.B. aus der Journal-Datei) als Eingabe verwendet. Zunächst werden die Fixationen für das erste Messzeitintervall (z.B. die ersten 30 Sekunden) der Aufnahme berechnet. Dieses Intervall entspricht der standardisierten Betrachtungslänge und kann als Ausgangspunkt für die zeitabhängige Analyse von Veränderungen dienen. Eine berechnete Fixation enthält Angaben über die Dauer und die Koordinaten in Blickfeld. Die Koordinatenpunkte der Fixationen innerhalb der ersten 30 Sekunden bilden eine Punktewolke in einem zwei-dimensionalen Koordinatensystem (Blickfeld X, Blickfeld Y).

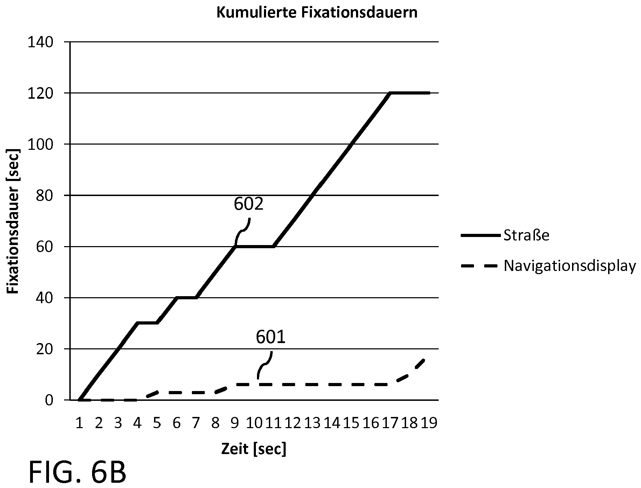

Die n Fixationen (schwarze Punkte) werden jetzt mit Hilfe einer k-Means-Analyse zu k (im Beispiel k=2) signifikant unterschiedlichen Clustern C1, C2 zugeordnet. Die k-Means-Analyse ist ein standardisiertes Instrument der Statistik und unterteilt die Cluster durch die Bildung und Verschiebung von k Clusterzentren (Mittelpunkte M1, M2) von denen ausgehend die euklidischen Distanzen zu allen umliegenden Fixationen berechnet werden. Zwei Cluster unterscheiden sich signifikant, wenn die durchschnittliche Distanz der Fixationen aus Cluster C1 zu dem Clusterzentrum M1 signifikant geringer ist, als zu Clusterzentrum C2 des Clusters M2 und umgekehrt. Die Anzahl $k \in [1, n]$ der Cluster ist dabei eine Eingabegröße in die Analyse und wird Iterativ von 1 bis n erhöht. Vorteilhafterweise wird die Anzahl k und Anordnung der Cluster gewählt, die die stärkste Heterogenität (Signifikanzniveau der Varianzanalyse zwischen den Distanzen) zwischen den Clustern und die stärkste Homogenität (kleiner Clusterumfang) innerhalb der Cluster ausweist. Im nächsten Schritt werden die Koordinaten der k Clustermittelpunkte Mk und die relativen Fixationshäufigkeiten auf die einzelnen Cluster berechnet. Die Fixationshäufigkeit auf ein Cluster beschreibt die Anzahl der Blicke auf dieses Cluster, also die Anzahl n der Fixationen. Für die Vergleichbarkeit zwischen den Clustern werden diese Fixationshäufigkeiten normiert, sodass relative Fixationshäufigkeiten entstehen, wie in FIG. 6B dargestellt.

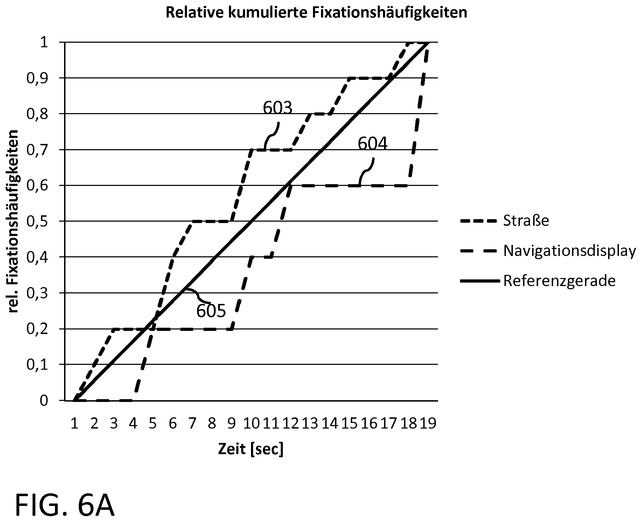

Die Kurven 601 bis 604 der Figuren 6A, 6B zeigen die Entwicklung der relativen Fixationshäufigkeiten und Fixationsdauern auf zwei Cluster (z.B. Straße und Navigationsdisplay) über die Zeit von den ersten 20 Sekunden einer Aufnahme. Immer wenn der zu einem Cluster gehörige Bereich angeschaut wird, steigt die jeweilige Kurve Fixationshäufigkeitskurve 603, 604 an und immer wenn es nicht angeschaut wird, verläuft die Kurve waagerecht. So kann die Regelmäßigkeit des Anschauens von einzelnen Clustern identifiziert werden. Zusätzlich ist eine Referenzgerade 605 mit einer Steigung von 45° eingezeichnet. Diese Gerade entspricht einer perfekt, regelmäßigen Fixationsverteilung. Schaut der Benutzer beispielsweise ein Cluster sechs Mal über einer Aufnahmedauer von 30 Sekunden an, so wäre dies perfekt regelmäßig, wenn er es jede fünf Sekunden anschaut. Als Klassifikation der Regelmäßigkeit wird die Abweichung zwischen den Clustern und der Referenzgerade 605 bestimmt. Die Cluster werden dann mit zunehmender Abweichung abwärts sortiert. Das Cluster mit der geringsten Abweichung wird als Informationsanker dieses Zeitintervalls t bezeichnet. Ein Informationsanker hat die Eigenschaft, dass er unabhängig von der Dauer und der Aufgabe sehr regelmäßig angeschaut wird, was das Einblenden von wichtigen, kurzen Hinweisen an genau diesen Stellen erleichtert, da es den Nutzer minimal ablenkt.

Neben den relativen Fixationshäufigkeiten in FIG. 6A können auch die kumulierten Fixationsdauern in FIG. 6B auf die Cluster berechnet werden. Die Kurven 601, 602 geben Aufschluss darüber wie lang ein Cluster bei jeder Fixation angeschaut wird (Steigungen der Kurve). Daraus wird die durchschnittliche Fixationsdauer berechnet. Das Cluster mit dem höchsten Wert, wird im Durchschnitt am längsten angeschaut und ist somit der Informationsfokus des Zeitintervalls t. Der Informationsfokus zeigt wo die Priorität der Benutzers liegt. Der Informationsfokus sollte immer zentral im Blickfeld liegen und die sekundären Elemente in geringem Abstand um den Informationsfokus herum. Nach der Berechnung der obenstehenden Größen wird das Betrachtungsintervall um ein Delta (z.B. fünf Sekunden) weiter geschoben und die gleichen Berechnungen für das neue Intervall angestellt. Dies wiederholt sich bis die gesamte Aufnahmedauer analysiert wurde. Jedes neu berechnete Betrachtungsintervall tx wird dabei mit dem jeweils vorangegangenen Intervall tx-1 hinsichtlich der berechneten Parameter verglichen, um so Veränderungen in der Strategie der Person zu identifizieren. Zunächst wird die Anzahl der Cluster k verglichen. Sollte sich k im Vergleich zum vorhergehenden Intervall verändert haben, so liegt ein Strategiewechsel zum Zeitpunkt tx vor, da entweder eine neue Informationsquelle berücksichtigt wurde oder eine nicht mehr berücksichtigt wurde.

Im nächsten Schritt wird überprüft, ob eine signifikante Verschiebung der Mittelpunkte der einzelnen Cluster vorliegt. Eine Verschiebung ist ebenfalls ein klares Indiz für eine Strategieänderung, da der Benutzer die gleichen Informationen an einer anderen Stelle sucht. Beispielsweise verschiebt sich bei einer Autofahrt das Cluster des betrachteten Straßenabschnitts nach unten, wenn der Benutzer von der Autobahn abfährt und in die Stadt einfährt. In diesem Moment sind die Dinge/Personen unmittelbar vor dem Fahrzeug wichtiger als diejenigen in weiterer Ferne.

Nachfolgend wird eine mögliche Abweichung des Informationsankers untersucht, die ebenfalls einen Strategiewechsel indiziert. Auch ein Wechsel des Informationsfokus kann für die Klassifikation eines Strategiewechsels zum Zeitpunkt tx herangezogen werden. Der mögliche Strategiewechsel kann nun zu einer Liste mit Zeitpunkten hinzugefügt werden. Dabei wird ebenfalls vermerkt, wie stark die Abweichung der Strategie zum vorher betrachteten Zeitintervall ist, indem die Anzahl der zutreffenden, möglichen Abweichungen protokolliert wird. Dieses Vorgehen wiederholt sich bis das Ende der Aufnahmedauer erreicht ist. Die Zeitpunkte der Strategiewechsel werden anschließend genutzt, um die Messzeitintervalle (Betrachtungszeitintervalle) für die Beanspruchungsmessung zu definieren. Sie ersetzen dabei den standardisierten Wert (z.B. von 30 Sekunden) und erlauben die Berechnung der Beanspruchung in Abhängigkeit der Vorgehensweise bzw. Blickstrategie des Benutzers, was so später die Ableitung von konkreten Gestaltungsmaßnahmen ermöglicht.

Im Anschluss werden die ausgewählten blickbasierten Beanspruchungsparameter (s. Tabelle 2) auf den Betrachtungsintervallen aus der Journal-Datei berechnet. Im Anschluss werden die bestimmten bewussten und unbewussten Parameter in die jeweiligen Beanspruchungsfunktionen $f(Pi)$ (ggf. gibt es eine Funktion für das Referenzsignal Leistung und eine Funktion für das Referenzsignal subjektives Empfinden) eingesetzt, um die Beanspruchung hinsichtlich der Leistung (und optional hinsichtlich des subjektiven Empfindens) auf den einzelnen Betrachtungsintervallen zuverlässig zu berechnen. Für den Fall, dass auch die mentale Effizienz als Referenzsignal eingesetzt wird, kann auch für dieses Referenzsignal eine entsprechende Beanspruchungsfunktion vorhanden sein, in die die jeweiligen Parameter eingesetzt werden.

Ausführungsformen der Erfindung können in Form von digitalen Schaltkreisen, Computerhardware, Firmware, Software oder in beliebigen Kombinationen davon implementiert werden. Die Erfindung kann weiterhin in Form eines Computerprogrammprodukts, z.B. eines Computerprogramms auf einem physischen Informationsträger (z.B., maschinenlesbares Speichermedium) implementiert werden, um von einer Datenverarbeitungsvorrichtung (z.B., programmierbarer Prozessor, Computer oder kommunikativ gekoppelte Computer) ausgeführt zu werden oder deren Betrieb zu steuern. Ein Computerprogrammprodukt wie beansprucht kann in einer beliebigen Programmiersprache erstellt sein, wobei auch kompilierte oder interpretierte Sprachen eingeschlossen sind. Es kann in beliebiger Form eingesetzt werden, beispielsweise als alleinstehendes Programm, Modul, Komponente, Unterprogramm oder als andere Einheit, die geeignet ist, um in einem Datenverarbeitungssystem benutzt zu werden. Das Computerprogramm kann von einem Computer ausgeführt werden oder aber auch durch mehrere über ein Kommunikationsnetzwerk miteinander verbundene Computer entweder an einem Ort oder über mehrere Orte verteilt. Ein computerimplementiertes Verfahren kann durch das Ausführen entsprechender Computerprogrammprodukte auf entsprechenden Datenverarbeitungsvorrichtungen ausgeführt werden.

Verfahrensschritte gemäß der Erfindung können von einem oder mehreren programmierbaren Prozessoren durch abarbeiten des Computerprogramms ausgeführt werden, um die erfindungsgemäßen Funktionen auszuführen, wobei Eingangsdaten verarbeitet werden und entsprechende Ausgangsdaten dabei erzeugt werden. Die Verfahrensschritte können auch durch spezielle Logikbausteine wie z.B. field programmable gate arrays (FPGA) oder application-specific integrated circuits (ASIC) ausgeführt werden.

Beispiele für Prozessoren, die geeignet zur Ausführung des Computerprogramms sind, beinhalten allgemeine oder spezialisierte Mikroprozessoren und jegliche Ein- oder Mehrprozessorlösung eines beliebigen digitalen Computers. Im allgemeinen erhält eine Prozessor Instruktionen und Daten von einem Read-Only Memory (ROM) oder Random Access Memory (RAM) oder von beiden. Die wesentlichen Elemente eines Computers sind mindestens ein Prozessor und ein oder mehrere Speichermedien, um Daten und Instruktionen zu speichern. Im Allgemeinen ist ein Computer auch mit einem oder mehreren Massenspeichermedien (z.B. magnetische, magneto-optische, optische oder solid state (SSD) Speichermedien) gekoppelt, um davon Daten zu empfangen oder Daten dort abzuspeichern. Solche Speichermedien können auch bei Bedarf (on demand) bereitgestellt werden oder über das Internet erreichbar sein (z.B. Cloud Computing). Geeignete Datenträger zum Abspeichern von Programminstruktionen und Daten umfassen alle Arten von nicht-flüchtigen Speicherelementen wie Halbleiterspeicherelemente (z.B., EPROM, EEPROM), Flash-Memory Vorrichtungen, magnetische oder magneto-optische Speichermedien, CD-ROM, DVD-ROM oder Blue-Ray Disks. Die Prozessor- und Speicherelemente können ergänzt werden durch spezielle Logikbausteine, oder auch Teil dieser sein.

Um die Interaktion mit dem Benutzer zu ermöglichen, kann die Erfindung auf einem Computer implementiert sein, der mindestens eine Ausgabevorrichtung (z.B., LCD Monitor, Lautsprecher, etc.) und mindestens eine Eingabevorrichtung (z.B. Tastatur, Touchscreen, Mikrofon, Zeigevorrichtung wie Maus oder Trackball) umfasst.

Der Erfindung kann auf einer Datenverarbeitungsvorrichtung implementiert werden, die eine Backend-Komponente (z.B. Datenserver) umfasst, oder eine Middleware-Komponente (z.B. Anwendungsserver), oder eine Frontend-Komponente (z.B. Client-Computer mit graphischem Benutzerinterface oder Webbrowser) worüber der Benutzer mit einer Ausführungsform der Erfindung interagieren kann, oder jegliche Kombination von Backend-, Middleware- und Frontendkomponenten.

Clientcomputer können auch mobile Endgeräte sein wie beispielsweise Smartphones, Tablet PCs oder jede beliebige tragbare Computervorrichtung. Die Komponenten des Systems können miteinander kommunikativ gekoppelt sein (z.B. mittels eines Kommunikationsnetz Werks wie Local Area Network LAN oder Wide Area Network WAN, Internet oder drahtlosen LAN oder Telekommunikationsnetzwerken).

Das Computersystem kann Clients und Server umfassen. Ein Client und ein Server sind im Allgemeinen physisch voneinander entfernt und interagieren über ein Kommunikationsnetzwerk. Die Beziehung zwischen Client und Server entsteht dabei durch Computerprogramme, die auf den jeweiligen Computern ausgeführt werden und die eine Client-Server Beziehung untereinander haben.

**Patentansprüche**

1. Computerimplementiertes Verfahren (1000) zur quantitativen Messung der mentalen Beanspruchung eines individuellen Benutzers (10) bei dessen Interaktion mit einem technischen System (200) über eine Benutzerschnittstelle, umfassend:

   Empfangen (1100) von einzelnen Parametersignalen für mehrere blickbasierte Beanspruchungsparameter (P1 to Pn) des individuellen Benutzers (10), die während der Bearbeitung mehrerer Kalibrierungsaufgaben durch den Benutzer (10) über jeweils zwei oder mehr Kalibrierungszeitintervalle hinweg erfasst werden, wobei die Kalibrierungsaufgaben zwei oder mehr verschiedene Aufgabenarten mit jeweils zwei oder mehr verschiedenen Anforderungsniveaus umfassen;

   Ermitteln (1200) von normierten Leistungsdaten als einzelne Referenzsignale für die jeweiligen Aufgaben aus den einzelnen Parametersignalen, wobei die Leistungsdaten ein Maß für die mentale Beanspruchung während der Bearbeitung der Kalibrierungsaufgaben darstellen;

   Erzeugen (1310) eines Gesamtreferenzsignals aus den einzelnen Referenzsignalen sowie der Gesamtparametersignale aus den einzelnen Parametersignalen für jeden erfassten Beanspruchungsparameter durch aneinanderreihen der jeweiligen Messergebnisse für alle Teilaufgaben der Kalibrierungsaufgaben;

   Auswählen (1300) der Beanspruchungsparameter, deren Gesamtparametersignale eine höhere Ähnlichkeit zum Gesamtreferenzsignal aufweisen als die Ähnlichkeit ihrer einzelnen Parametersignale zu den jeweiligen einzelnen Referenzsignalen für die einzelnen Ausprägungen von Aufgabenart, Anforderungsniveau und Zeitintervall, wobei die jeweiligen Ähnlichkeiten jeweils über eine Procrustes-Analyse ermittelt werden;

   Ermitteln (1400) von mindestens einem drift-freien Beanspruchungsparameters (Pi) mit der höchsten Adaptionslatenz unter den ausgewählten Beanspruchungsparametern;

   Generieren (1500) einer individuellen Beanspruchungsfunktion (f(P(i)) für den Benutzer durch Überführen von mindestens einem der ermittelten drift-freien Beanspruchungsparameter (Pi) in eine individuelle lineare Funktion mittels linearer Regression aus den während der Kalibrierungsaufgabe für den mindestens einen ermittelten drift-freien Beanspruchungsparameter erfassten Leistungsdaten;

   Erfassen (1600) des mindestens einen ermittelten drift-freien Beanspruchungsparameters während der Interaktion des individuellen Benutzers mit dem technischen System über mehrere Messzeitintervalle; und

   Ableiten (1700) der Leistungsdaten pro Messzeitintervall als mentale Beanspruchung mittels der individuellen Beanspruchungsfunktion.

2. Verfahren nach Anspruch 1, weiter umfassend Schritte für das Bestimmen (1800) der mentalen Beanspruchung in Abhängigkeit einer Blickstrategie:

   Erfassen (1820) von Fixationskoordinaten, Fixationshäufigkeit und Fixationsdauer des individuellen Benutzers während der Interaktion mit dem technischen System;

   Ermitteln (1840) von Indikatoren für die Blickstrategie des individuellen Benutzers mit einer Kombination aus einer Clusteranalyse der Fixationskoordinaten und einer Analyse der Fixationshäufigkeit und -dauer;

   Bestimmen (1860) der Grenzen der Messzeitintervalle als Zeitpunkte von Blickstrategiewechseln mittels Veränderungen in den Ergebnissen der Clusteranalyse der Fixationskoordinaten oder der Analyse von Fixationshäufigkeit und Fixationsdauer; und

   Ableiten (1880) der mentalen Beanspruchung des individuellen Benutzers in Abhängigkeit der angewandten Blickstrategie.

3. Verfahren nach einem der vorherigen Ansprüche, wobei das Auswählen (1300) der Beanspruchungsparameter weiterhin folgende Schritte umfasst:

Ermitteln (1330) einer Ähnlichkeit für jedes der Gesamtparametersignale mit dem Gesamtreferenzsignal über die Procrustes-Analyse;

Aufteilen (1350) der Gesamtparametersignale und des Gesamtreferenzsignals nach den Ausprägungen der Aufgabenarten, der Anforderungsniveaus und der einzelnen Zeitintervalle;

Durchführen (1370) der Procrustes-Analyse für jede Ausprägung, zwischen den einzelnen Parametersignalen und den einzelnen Referenzsignalen; und

Vergleichen (1390) der Ähnlichkeiten der einzelnen Parametersignale zu den einzelnen Referenzsignalen mit der Ähnlichkeit der Gesamtparametersignale zum Gesamtreferenzsignal.

4. Verfahren nach einem der vorherigen Ansprüche, wobei das Ermitteln (1400) des mindestens einen drift-freien Beanspruchungsparameters mit der höchsten Adaptionslatenz weiterhin umfasst:

Bestimmen (1420) einer Adaptionslatenz für jeden gefilterten Beanspruchungsparameter mittels einer Moving-Correlation-Analyse und vergleichen der mittleren Adaptionslatenz von bewussten Beanspruchungsparametern mit der mittleren Adaptionslatenz von unbewussten Beanspruchungsparametern und selektieren der gefilterten Beanspruchungsparameter der Kategorie mit der höheren mittleren Adaptionslatenz;

Bestimmen (1440) von drift-freien selektierten Beanspruchungsparametern, die im Betrachtungsbereich des Referenzsignals nicht von dem Referenzsignal abdriften, und

Ermitteln (1460) des drift-freien Beanspruchungsparameters mit der höchsten Adaptionslatenz.

5. Verfahren nach einem der vorherigen Ansprüche, wobei die Nebenaufgaben arithmetische und visuelle Aufgaben umfassen.

6. Verfahren nach Anspruch 5, wobei für visuelle Aufgaben zusätzlich zu den Leistungsdaten die Reaktionszeiten erfasst werden und für die Ermittlung der Referenzsignale verwendet werden.

7. Verfahren nach einem der vorherigen Ansprüche, wobei zusätzlich zu den Leistungsdaten das subjektive Beanspruchungsempfinden (124) des individuellen Benutzers (10) erfasst wird und für die Ermittlung der Referenzsignale (121) verwendet wird.

8. Verfahren nach einem der vorherigen Ansprüche, wobei Beanspruchungsparameter die einen Indikator für einen bewussten Verarbeitungsprozess repräsentieren einer der folgenden bewussten Parametergruppen zugeordnet sind:
Fixationsdauer, Sakkadenlänge, Nearest Neighbor Index; und
wobei Beanspruchungsparameter die einen Indikator für einen unbewussten Verarbeitungsprozess repräsentieren einer der folgenden bewussten Parametergruppen zugeordnet sind:
Blinzelrate, Blinzeldauer, PERCLOS, Pupillendurchschnittsvariabilität.

9. Computerprogrammprodukt umfassend Instruktionen, welche beim Laden in mindestens ein Speichermodul eines Computers und Abarbeiten durch mindestens einen Prozessor des Computers das computerimplementierte Verfahren nach einem der vorherigen Ansprüche ausführen.

10. Computersystem (100) zur quantitativen Messung der mentalen Beanspruchung eines individuellen Benutzers (10) bei dessen Interaktion mit einem technischen System (200) über eine Benutzerschnittstelle, umfassend :

Ein erstes Erfassungsmodul (110) konfiguriert zum Empfangen von einzelnen Parametersignalen (P1 - Pn) für mehrere blickbasierte Beanspruchungsparameter des individuellen Benutzers (10), die während der Bearbeitung mehrerer Kalibrierungsaufgaben über jeweils zwei oder mehr Kalibrierungszeitintervalle hinweg erfasst werden, wobei die Kalibrierungsaufgaben zwei oder mehr verschiedene Aufgabenarten mit jeweils zwei oder mehr verschiedenen Anforderungsniveaus umfassen;
Ein Auswertemodul (120) konfiguriert zum:

Ermitteln von normierten Leistungsdaten (122) als einzelne Referenzsignale (121) für die jeweiligen Aufgaben aus den einzelnen Parametersignalen (P1 - Pn), wobei die Leistungsdaten ein Maß für die mentale Beanspruchung des Benutzers während der Bearbeitung der Kalibrierungsaufgaben darstellen;

Erzeugen (1310) eines Gesamtreferenzsignals aus den einzelnen Referenzsignalen sowie der Gesamtparametersignale aus den einzelnen Parametersignalen für jeden erfassten Beanspruchungsparameter durch aneinanderreihen der jeweiligen Messergebnisse für alle Teilaufgaben der Kalibrierungsaufgaben;

Auswählen der Beanspruchungsparameter, deren Gesamtparametersignale eine höhere Ähnlichkeit zum Gesamtreferenzsignal aufweisen als die Ähnlichkeit ihrer einzelnen Parametersignale zu den jeweiligen einzelnen Referenzsignalen für die einzelnen Ausprägungen von Aufgabenart, Anforderungsniveau und Zeitintervall, wobei die jeweiligen Ähnlichkeiten jeweils über eine Procrustes-Analyse ermittelt werden;

Ermitteln von mindestens einem drift-freien Beanspruchungsparameter (Pi) mit der höchsten Adaptionslatenz unter den ausgewählten Beanspruchungsparametern; und

Generieren einer individuellen Beanspruchungsfunktion (f(Pi)) für den Benutzer (10) durch Überführen von mindestens einem der ermittelten drift-freien Beanspruchungsparameter (Pi) in eine individuelle lineare Funktion mittels linearer Regression aus den während der Kalibrierungsaufgabe für den mindestens einen der ermittelten drift-freien Beanspruchungsparameter (Pi) erfassten Leistungsdaten (122);

Ein zweites Erfassungsmodul (130) konfiguriert zum Erfassen des mindestens einen ermittelten drift-freien Beanspruchungsparameters (Pi) während der Interaktion des individuellen Benutzers mit dem technischen System (200) über mehrere Messzeitintervalle; und

Ein Analysemodul (140) konfiguriert zum Ableiten der Leistungsdaten pro Messzeitintervall als mentale Beanspruchung mittels der individuellen Beanspruchungsfunktion (fP(i)).

11. Computersystem nach Anspruch 10, mit dem zweiten Erfassungsmodul (130) weiterhin konfiguriert zum:

Erfassen von Fixationskoordinaten (F1), Fixationshäufigkeit (F2) und Fixationsdauer (F3) des individuellen Benutzers (10) während der Interaktion mit dem technischen System (200);

Und das Analysemodul (140) weiterhin konfiguriert zum:

Ermitteln von Indikatoren für die Blickstrategie des individuellen Benutzers mit einer Kombination aus einer Clusteranalyse der Fixationskoordinaten (F1) und einer Analyse der Fixationshäufigkeit (F2) und Fixationsdauer (F3);

Bestimmen der Grenzen der Messzeitintervalle als Zeitpunkte von Blickstrategiewechseln mittels Veränderungen in den Ergebnissen der Clusteranalyse der Fixationskoordinaten oder der Analyse von Fixationshäufigkeit und -dauer; und

Ableiten der mentalen Beanspruchung des individuellen Benutzers in Abhängigkeit der angewandten Blickstrategie.

12. Computersystem nach einem der Ansprüche 10 bis 11, mit dem Auswertemodul weiterhin konfiguriert zum Auswählen der Beanspruchungsparameter mit den folgende Schritten:

Ermitteln einer Ähnlichkeit für jedes der Gesamtparametersignale mit dem Gesamtreferenzsignal über die Procrustes-Analyse;

Aufteilen der Gesamtparametersignale und des Gesamtreferenzsignals nach den Ausprägungen der Aufgabenarten, der Anforderungsniveaus und der einzelnen Zeitintervalle;

Durchführen der Procrustes-Analyse für jede Ausprägung, zwischen den einzelnen Parametersignalen und den einzelnen Referenzsignalen; und

Vergleichen der Ähnlichkeiten der einzelnen Parametersignale zu den einzelnen Referenzsignalen mit der Ähnlichkeit der Gesamtparametersignale zum Gesamtreferenzsignal.

13. Computersystem nach einem der Ansprüche 10 bis 12, mit dem Auswertemodul weiterhin konfiguriert zum Ermitteln des drift-freien Beanspruchungsparameters mit der höchsten Adaptionslatenz durch:

Bestimmen einer Adaptionslatenz für jeden gefilterten Beanspruchungsparameter mittels einer Moving-Correlation-Analyse und vergleichen der mittleren Adaptionslatenz von bewussten Beanspruchungsparametern mit der mittleren Adaptionslatenz von unbewussten Beanspruchungsparametern und selektieren der gefilterten Beanspruchungsparameter der Kategorie mit der höheren mittleren Adaptionslatenz;

Bestimmen von drift-freien selektierten Beanspruchungsparametern, die im Betrachtungsbereich des Referenzsignals nicht von dem Referenzsignal abdriften, und

Ermitteln des drift-freien Beanspruchungsparameters mit der höchsten Adaptionslatenz.

**Claims**

1. Computer-implemented method (1000) for quantitatively measuring the mental workload of an individual user (10) interacting with a technical system (200) via a user interface, comprising:

   receiving (1100) individual parameter signals for a plurality of gaze-based workload parameters (P1 to Pn) of the individual user (10) which are detected while multiple calibration tasks are being processed by the user (10) over two or more calibration time intervals, wherein the calibration tasks comprise two or more different types of tasks, each having two or more different requirement levels;

   ascertaining (1200) normalized performance data as individual reference signals for the various tasks from the individual parameter signals, wherein the performance data are a measure of the mental workload during processing of the calibration tasks;

   generating (1310) an overall reference signal from the individual reference signals and the overall parameter signals from the individual parameter signals for each detected workload parameter by juxtaposing the respective measurement results for all subtasks of the calibration tasks;

   selecting (1300) the workload parameters of which the overall parameter signals have a greater similarity to the overall reference signal than the similarity between the individual parameter signals thereof and the respective individual reference signals for the individual characteristics of the task type, requirement level, and time interval, wherein the respective similarities are determined via a Procrustes analysis;

   ascertaining (1400) at least one drift-free workload parameter (Pi) having the highest adaptation latency among the selected workload parameters;

   generating (1500) an individual workload function (f(P(i)) for the user by translating at least one of the ascertained drift-free workload parameters (Pi) into an individual linear function by means of linear regression from the performance data detected during the calibration task for the at least one ascertained drift-free workload parameter;

   detecting (1600) the at least one ascertained drift-free workload parameter during the interaction of the individual user with the technical system over a plurality of measurement time intervals; and

   deriving (1700) the performance data per measurement time interval as mental workload by means of the individual workload function.

2. Method according to claim 1, further comprising steps for determining (1800) the mental workload on the basis of a gaze strategy:

   detecting (1820) fixation coordinates, fixation frequency and fixation duration of the individual user during interaction with the technical system;

   ascertaining (1840) indicators for the gaze strategy of the individual user by means of a combination of a cluster analysis of the fixation coordinates and an analysis of the fixation frequency and duration;

   determining (1860) the limits of the measurement time intervals as times of gaze strategy changes by means of changes in the results of the cluster analysis of the fixation coordinates or the analysis of fixation frequency and fixation duration; and

   deriving (1880) the mental workload of the individual user on the basis of the applied gaze strategy.

3. Method according to either of the preceding claims, wherein selecting (1300) the workload parameters further comprises the following steps:

   ascertaining (1330) a similarity between each of the overall parameter signals and the overall reference signal via the Procrustes analysis;

   dividing up (1350) the overall parameter signals and the overall reference signal according to the characteristics of the task types, the requirement levels and the individual time intervals;

   performing (1370) the Procrustes analysis for each characteristic, between the individual parameter signals and the individual reference signals; and

   comparing (1390) the similarities between the individual parameter signals and the individual reference signals with the similarity between the overall parameter signals and the overall reference signal.

4. Method according to any of the preceding claims, wherein ascertaining (1400) the at least one drift-free workload parameter having the highest adaptation latency further comprises:

   determining (1420) an adaptation latency for each filtered workload parameter by means of a moving correlation analysis and comparing the mean adaptation latency of conscious workload parameters with the mean adaptation latency of unconscious workload parameters and selecting the filtered workload parameters of the category having the higher mean adaptation latency;

determining (1440) drift-free selected workload parameters that do not drift away from the reference signal in the observation range of the reference signal, and

ascertaining (1460) the drift-free workload parameter having the highest adaptation latency.

5. Method according to any of the preceding claims, wherein the ancillary tasks include arithmetic and visual tasks.

6. Method according to claim 5, wherein, in addition to the performance data, the response times for visual tasks are detected and used for ascertaining the reference signals.

7. Method according to any of the preceding claims, wherein, in addition to the performance data, the subjective workload perception (124) of the individual user (10) is detected and used for ascertaining the reference signals (121).

8. Method according to any of the preceding claims, wherein workload parameters which are an indicator of a conscious processing process are associated with one of the following conscious parameter groups:

fixation duration, saccade length, nearest neighbor index; and

wherein workload parameters which are an indicator for an unconscious processing process are associated with one of the following conscious parameter groups:

blink rate, blink duration, PERCLOS, average pupil variability.

9. Computer program product comprising instructions which, when loaded into at least one memory module of a computer and processed by at least one processor of the computer, execute the computer-implemented method according to any of the preceding claims.

10. Computer system (100) for quantitatively measuring the mental workload of an individual user (10) interacting with a technical system (200) via a user interface, comprising

a first detection module (110) configured to receive individual parameter signals (P1 - Pn) for a plurality of gaze-based workload parameters of the individual user (10) which are detected while multiple calibration tasks are being processed over two or more calibration time intervals, wherein the calibration tasks comprise two or more different types of tasks, each having two or more different requirement levels;

an evaluation module (120) configured to:

ascertain normalized performance data (122) as individual reference signals (121) for the various tasks from the individual parameter signals (P1 - Pn), wherein the performance data are a measure of the mental workload of the user during processing of the calibration tasks;

generate (1310) an overall reference signal from the individual reference signals and the overall parameter signals from the individual parameter signals for each detected workload parameter by juxtaposing the respective measurement results for all subtasks of the calibration tasks;

select the workload parameters of which the overall parameter signals have a greater similarity to the overall reference signal than the similarity between the individual parameter signals thereof and the respective individual reference signals for the individual characteristics of task type, requirement level and time interval, wherein the respective similarities are ascertained in each case via a Procrustes analysis;

ascertain at least one drift-free workload parameter (Pi) having the highest adaptation latency among the selected workload parameters; and

generate an individual workload function (f(Pi)) for the user (10) by translating at least one of the ascertained drift-free workload parameters (Pi) into an individual linear function by means of linear regression from the performance data (122) detected during the calibration task for the at least one of the ascertained drift-free workload parameters (Pi);

a second detection module (130) configured to detect the at least one ascertained drift-free workload parameter (Pi) during the interaction of the individual user with the technical system (200) over a plurality of measurement time intervals; and

an analysis module (140) configured to derive the performance data per measurement time interval as mental workload by means of the individual workload function (fP(i)).

**11.** Computer system according to claim 10, comprising the second detection module (130) that is further configured to: detect fixation coordinates (F1), fixation frequency (F2) and fixation duration (F3) of the individual user (10) during interaction with the technical system (200); and the analysis module (140) is further configured to:

ascertain indicators for the gaze strategy of the individual user by means of a combination of a cluster analysis of the fixation coordinates (F1) and an analysis of the fixation frequency (F2) and fixation duration (F3); determine the limits of the measuring time intervals as times of gaze strategy changes by means of changes in the results of the cluster analysis of the fixation coordinates or the analysis of fixation frequency and duration; and derive the mental workload of the individual user on the basis of the applied gaze strategy.

**12.** Computer system according to either claim 10 or claim 11, comprising the evaluation module that is further configured to select the workload parameters by means of the following steps:

ascertaining a similarity between each of the overall parameter signals and the overall reference signal via the Procrustes analysis; dividing up the overall parameter signals and the overall reference signal according to the characteristics of the task types, the requirement levels and the individual time intervals; performing the Procrustes analysis for each characteristic, between the individual parameter signals and the individual reference signals; and comparing the similarities between the individual parameter signals and the individual reference signals with the similarity between the overall parameter signals and the overall reference signal.

**13.** Computer system according to any of claims 10 to 12, comprising the evaluation module that is further configured to ascertain the drift-free workload parameter having the highest adaptation latency by:

determining an adaptation latency for each filtered workload parameter by means of a moving correlation analysis and comparing the mean adaptation latency of conscious workload parameters with the mean adaptation latency of unconscious workload parameters and selecting the filtered workload parameters of the category having the higher mean adaptation latency; determining drift-free selected workload parameters that do not drift away from the reference signal in the observation range of the reference signal, and ascertaining the drift-free workload parameter having the highest adaptation latency.

**Revendications**

**1.** Procédé mis en œuvre par ordinateur (1000), destiné à mesurer quantitativement la contrainte mentale d'un utilisateur individuel (10) interagissant avec un système technique (200) par l'intermédiaire d'une interface utilisateur, comprenant :

la réception (1100) de signaux de paramètres individuels pour une pluralité de paramètres de contrainte (P1 à Pn) basés sur le regard de l'utilisateur individuel (10), détectés au cours du traitement de multiples tâches d'étalonnage par l'utilisateur (10) sur au moins deux intervalles de temps d'étalonnage, les tâches d'étalonnage comprenant au moins deux types de tâches différents présentant chacun au moins deux niveaux d'exigence différents ; la définition (1200) de données de performance normalisées comme étant des signaux de référence individuels pour les tâches respectives à partir des signaux de paramètres individuels, les données de performance étant une mesure de la contrainte mentale pendant le traitement des tâches d'étalonnage ; la génération (1310) d'un signal de référence global à partir des signaux de référence individuels, et des signaux de paramètres globaux à partir des signaux de paramètres individuels pour chaque paramètre de contrainte détecté en juxtaposant les résultats de mesure respectifs pour toutes les sous-tâches des tâches d'étalonnage ; la sélection (1300) des paramètres de contrainte dont les signaux de paramètres globaux présentent une similitude plus élevée avec le signal de référence global que la similitude de leurs signaux de paramètres individuels avec les signaux de référence individuels respectifs pour les formes individuelles de types de tâche, de niveaux d'exigence et d'intervalles de temps, les similitudes respectives étant déterminées à l'aide d'une analyse de Procruste ;

de détermination (1400) d'au moins un paramètre de contrainte (Pi) sans dérive présentant la latence d'adaptation la plus élevée parmi les paramètres de contrainte sélectionnés ;

la génération (1500) d'une fonction de contrainte (f(P(i)) individuelle pour l'utilisateur en transférant au moins l'un des paramètres de contrainte (Pi) sans dérive déterminés dans une fonction linéaire individuelle au moyen de la régression linéaire à partir des données de performance recueillies pendant la tâche d'étalonnage pour l'au moins un paramètre de contrainte sans dérive déterminé ;

de détection (1600) de l'au moins un paramètre de contrainte sans dérive déterminé lors de l'interaction de l'utilisateur individuel avec le système technique sur une pluralité d'intervalles de temps de mesure ; et

de dérivation (1700) des données de performance par intervalle de temps de mesure comme contrainte mentale au moyen de la fonction de contrainte individuelle.

2. Procédé selon la revendication 1, comprenant en outre les étapes, pour la détermination (1800) de la contrainte mentale en fonction d'une stratégie de regard :

de détection (1820) des coordonnées de fixation, de la fréquence de fixation et de la durée de fixation de l'utilisateur individuel lors de l'interaction avec le système technique ;

de détermination (1840) des indicateurs de la stratégie de regard de l'utilisateur individuel avec une combinaison d'une analyse en grappes des coordonnées de fixation et d'une analyse de la fréquence et de la durée de fixation ;

de détermination (1860) des limites des intervalles de temps de mesure comme moments de changement de stratégie de regard par la modification des résultats de l'analyse par grappes des coordonnées de fixation ou de l'analyse de la fréquence et de la durée de fixation ; et

de dérivation (1880) de la contrainte mentale de l'utilisateur individuel en fonction de la stratégie de regard utilisée.

3. Procédé selon l'une des revendications précédentes, dans lequel la sélection (1300) du paramètre de contrainte comprend en outre les étapes :

de détermination (1330) d'une similitude pour chacun des signaux de paramètres globaux avec le signal de référence global à l'aide d'une analyse de Procruste ;

de division (1350) des signaux de paramètres globaux et du signal de référence global en fonction des formes de types de tâches, des niveaux d'exigence et des intervalles de temps individuels ;

de réalisation (1370) de l'analyse de Procruste, pour chaque forme, entre les signaux de paramètres individuels et les signaux de référence individuels ; et

de comparaison (1390) des similitudes entre les signaux de paramètres individuels et les signaux de référence individuels avec la similitude entre les signaux de paramètres globaux et le signal de référence global.

4. Procédé selon l'une des revendications précédentes, dans lequel la détermination (1400) de l'au moins un paramètre de contrainte sans dérive présentant la latence d'adaptation la plus élevée comprend en outre :

la détermination (1420) d'une latence d'adaptation pour chaque paramètre de contrainte filtré à l'aide d'une analyse de corrélation mobile, la comparaison de la latence d'adaptation moyenne des paramètres de contrainte conscients à la latence moyenne d'adaptation de paramètres de contrainte inconscients, et la sélection du paramètre de contrainte filtré de la catégorie présentant la latence d'adaptation moyenne la plus élevée ;

la détermination (1440) de paramètres de contrainte sélectionnés sans dérive qui ne s'éloignent pas du signal de référence dans le champ d'observation du signal de référence, et

la détermination (1460) du paramètre de contrainte sans dérive présentant la latence d'adaptation la plus élevée.

5. Procédé selon l'une des revendications précédentes, dans lequel les tâches secondaires comprennent des tâches arithmétiques et visuelles.

6. Procédé selon la revendication 5, dans lequel, pour les tâches visuelles, outre les données de performance, les temps de réponse sont détectés et utilisés pour la détermination des signaux de référence.

7. Procédé selon l'une des revendications précédentes, dans lequel, outre les données de performance, la perception subjective de contrainte (124) de l'utilisateur individuel (10) est détectée et est utilisée pour la détermination des signaux de référence (121).

8. Procédé selon l'une des revendications précédentes, dans lequel les paramètres de contrainte représentant un indicateur d'un processus de traitement conscient associé à l'un des groupes de paramètres de conscience suivants :

durée de fixation, longueur de saccade, index du plus proche voisin ; et
dans lequel les paramètres de contrainte représentant un indicateur d'un processus de traitement inconscient sont associés à l'un des groupes de paramètres de conscience suivants :
vitesse de clignement, durée de clignement, pourcentage de fermeture des yeux (PERCLOS), variabilité moyenne des pupilles.

9. Produit de programme informatique comprenant des instructions qui, lorsqu'elles sont chargées dans au moins un module de mémoire d'un ordinateur et exécutées par au moins un processeur de l'ordinateur, exécutent le procédé mis en oeuvre par ordinateur selon l'une des revendications précédentes.

10. Système informatique (100) destiné à mesurer quantitativement la contrainte mentale d'un utilisateur individuel (10) interagissant avec un système technique (200) par l'intermédiaire d'une interface utilisateur, comprenant

un premier module de détection (110) configuré pour recevoir des signaux de paramètres (P1 à Pn) individuels pour une pluralité de paramètres de contrainte basés sur le regard de l'utilisateur individuel (10), détectés au cours du traitement de multiples tâches d'étalonnage sur au moins deux intervalles de temps d'étalonnage, les tâches d'étalonnage comprenant au moins deux types de tâches différents présentant chacun au moins deux niveaux d'exigence différents ;
un module d'évaluation (120) configuré pour :

déterminer des données de performance (122) normalisées comme étant des signaux de référence (121) individuels pour les tâches respectives à partir des signaux de paramètres (P1 à Pn) individuels, les données de performance étant une mesure de la contrainte mentale de l'utilisateur pendant le traitement des tâches d'étalonnage ;
générer (1310) un signal de référence global à partir des signaux de référence individuels, et des signaux de paramètres globaux à partir des signaux de paramètres individuels pour chaque paramètre de contrainte détecté en juxtaposant les résultats de mesure respectifs pour toutes les sous-tâches des tâches d'étalonnage ;
sélectionner les paramètres de contrainte dont les signaux de paramètres globaux présentent une similitude plus élevée avec le signal de référence global que la similitude de leurs signaux de paramètres individuels avec les signaux de référence individuels respectifs pour les formes individuelles de types de tâche, de niveaux d'exigence et d'intervalles de temps, les similitudes respectives étant déterminées à l'aide d'une analyse de Procruste ;
déterminer au moins un paramètre de contrainte (Pi) sans dérive présentant la latence d'adaptation la plus élevée parmi les paramètres de contrainte sélectionnés ; et
générer une fonction de contrainte (f(Pi)) individuelle pour l'utilisateur (10) en transférant au moins l'un des paramètres de contrainte (Pi) sans dérive déterminés dans une fonction linéaire individuelle au moyen de la régression linéaire à partir des données de performance (122) recueillies pendant la tâche d'étalonnage pour l'au moins un paramètre de contrainte (Pi) sans dérive déterminé ;

un second module de détection (130) configuré pour détecter l'au moins un paramètre de contrainte (Pi) sans dérive déterminé lors de l'interaction de l'utilisateur individuel avec le système technique (200) sur une pluralité d'intervalles de temps de mesure ; et
un module d'analyse (140) configuré pour dériver les données de performance par intervalle de temps de mesure comme contrainte mentale au moyen de la fonction de contrainte (fP(i)) individuelle.

11. Système informatique selon la revendication 10, dans lequel le second module de détection (130) est en outre configuré pour :
détecter des coordonnées de fixation (F1), la fréquence de fixation (F2) et de la durée de fixation (F3) de l'utilisateur individuel (10) lors de l'interaction avec le système technique (200) ;
et le module d'analyse (140) est en outre configuré pour :

déterminer des indicateurs de la stratégie de regard de l'utilisateur individuel en combinant une analyse en grappes des coordonnées de fixation (F1) et une analyse de la fréquence de fixation (F2) et de la durée de fixation (F3) ;
déterminer les limites des intervalles de temps de mesure comme moments de changement de stratégie de regard en modifiant les résultats de l'analyse par grappes des coordonnées de fixation ou de l'analyse de la fréquence et de la durée de fixation ; et

dériver la contrainte mentale de l'utilisateur individuel en fonction de la stratégie de regard utilisée.

12. Système informatique selon l'une des revendications 10 à 11, dans lequel le module d'évaluation est en outre configuré pour sélectionner les paramètres de contrainte en exécutant les étapes :

de détermination d'une similitude pour chacun des signaux de paramètres globaux avec le signal de référence global à l'aide d'une analyse de Procruste ;

de division des signaux de paramètres globaux et du signal de référence global en fonction des formes de types de tâches, de niveaux d'exigence et d'intervalles de temps individuels ;

de réalisation de l'analyse de Procruste, pour chaque forme, entre les signaux de paramètres individuels et les signaux de référence individuels ; et

de comparaison des similitudes entre les signaux de paramètres individuels et les signaux de référence individuels avec la similitude entre les signaux de paramètres globaux et le signal de référence global.

13. Système informatique selon l'une des revendications 10 à 12, dans lequel le module d'évaluation est en outre configuré pour déterminer le paramètre de contrainte sans dérive présentant la latence d'adaptation la plus élevée :

en déterminant une latence d'adaptation pour chaque paramètre de contrainte filtré à l'aide d'une analyse de corrélation mobile, en comparant la latence d'adaptation moyenne des paramètres de contrainte conscients à la latence moyenne d'adaptation de paramètres de contrainte inconscients, et en sélectionnant le paramètre de contrainte filtré de la catégorie présentant la latence d'adaptation moyenne la plus élevée ;

en déterminant des paramètres de contrainte sélectionnés sans dérive qui ne s'éloignent pas du signal de référence dans le champ d'observation du signal de référence, et

en déterminant le paramètre de contrainte sans dérive présentant la latence d'adaptation la plus élevée.

**FIG. 1**

1000

| Empfangen von einzelnen Parametersignalen für blickbasierte Beanspruchungsparameter während der Bearbeitung mehrerer Kalibrierungsaufgaben | 1100 |

| Ermitteln von normierten Leistungsdaten als Referenzsignale aus den einzelnen Parametersignalen | 1200 |

| Auswählen der Beanspruchungsparameter mit höherer Ähnlichkeit der Gesamtparametersignale zum Gesamt-referenzsignal als die Ähnlichkeit der entsprechenden Einzelsignale bezüglich Ausprägungen von Aufgabenart, Anforderungsniveau und Zeitintervall | 1300 |

| Ermitteln von mindestens einem drift-freien Beanspruchungsparameters mit der höchsten Adaptionslatenz unter den ausgewählten Beanspruchungsparametern | 1400 |

| Generieren einer individuellen Beanspruchungsfunktion für den Benutzer | 1500 |

| Erfassen des ermittelten drift-freien Beanspruchungsparameters während der Interaktion des individuellen Benutzers mit einem technischen System | 1600 |

| Ableiten der Leistungsdaten pro Messzeitintervall als mentale Beanspruchung mittels der individuellen Beanspruchungsfunktion | 1700 |

| Bestimmen der mentalen Beanspruchung in Abhängigkeit der Blickstrategie | 1800 |

# FIG. 2A

1800

Erfassen von Fixationskoordinaten, Fixationshäufigkeit und Fixationsdauer

1820

Ermitteln von Indikatoren für die Blickstrategie des individuellen Benutzers

1840

Bestimmen der Grenzen der Messzeitintervalle als Zeitpunkte von Blickstrategiewechseln

1860

Ableiten der mentalen Beanspruchung des individuellen Benutzers in Abhängigkeit der angewandten Blickstrategie

1880

## FIG. 2B

1300

Erzeugen eines Gesamtreferenzsignals sowie von Gesamtparametersignalen

1310

Ermitteln einer Ähnlichkeit für jedes der Gesamtparametersignale mit dem Gesamtreferenzsignal

1330

Aufteilen der Gesamtparametersignale und des Gesamtreferenzsignals nach Ausprägungen der Aufgabenarten, der Anforderungsniveaus und der einzelnen Zeitintervalle

1350

Durchführen der Procrustes-Analyse für jede Ausprägung, zwischen den einzelnen Parametersignalen und den einzelnen Referenzsignalen

1370

Vergleichen der Ähnlichkeiten der einzelnen Parametersignale zu den einzelnen Referenzsignalen mit der Ähnlichkeit der Gesamtparametersignale zum Gesamtreferenzsignal

1390

## FIG. 2C

<u>1400</u>

| Bestimmen einer Adaptionslatenz für jeden gefilterten Beanspruchungsparameter |
|---|

~1420

| Bestimmen von drift-freien Beanspruchungsparametern |
|---|

~1440

| Ermitteln des drift-freien Beanspruchungsparameters mit der höchsten Adaptionslatenz |
|---|

~1460

# FIG. 2D

<u>300</u>

# FIG. 3A

<u>300</u>

# FIG. 3B

**Beanspruchungsfunktion: Leistung**

FIG. 4A

**Beanspruchungsfunktion: Subjektives Empfinden**

FIG. 4B

FIG. 5

**Relative kumulierte Fixationshäufigkeiten**

FIG. 6A

**Kumulierte Fixationsdauern**

FIG. 6B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014159793 A1

**In der Beschreibung aufgeführte Nicht-Patentliteratur**

- **PACKEBUSCH, L.** Psychische Belastung und Beanspruchung - Normung für die Praxis. *Wirtschaftspsychologie aktuell,* 2003, vol. 4, 32-36
- **NIEDERL, T.** *Deutscher Psychologen Verlag GmbH,* 2007
- Psychophysiologie mentaler Beanspruchung. **MANZEY, D.** Enyklopädie der Psychologie. 1998, vol. 5, 799-864
- **STRÄTER, O.** *Cognition and Safety: An integrated approach to systems design and assessment,* 2005
- **WICKENS, C. D. et al.** Engineering psychology and human performance. 2013
- **OSIS, S. T. et al.** A novel method tp evaluate error in anatomical marker placement using a modified generalized Procrustes analysis. *Computer methods in biomechanics and biomedical engineering,* 2015, vol. 10, 1108-1116